(19)

Europäisches
Patentamt

European
Patent Office

Office européen
des brevets

(11)　EP 3 074 551 B1

## (12)　EUROPEAN PATENT SPECIFICATION

(45) Date of publication and mention
of the grant of the patent:
**22.11.2023 Bulletin 2023/47**

(21) Application number: **14863426.4**

(22) Date of filing: **25.11.2014**

(51) International Patent Classification (IPC):
*G01N 33/487* (2006.01)　　*C23C 18/28* (2006.01)
*C25D 11/16* (2006.01)　　*D01F 11/16* (2006.01)
*B82Y 30/00* (2011.01)　　*B82Y 40/00* (2011.01)
*C23C 16/56* (2006.01)　　*C01B 32/186* (2017.01)
*C23C 16/02* (2006.01)　　*C23C 16/26* (2006.01)

(52) Cooperative Patent Classification (CPC):
**G01N 33/48721; C01B 32/186; C23C 16/0281;
C23C 16/26; C23C 16/56; B82Y 30/00; B82Y 40/00**

(86) International application number:
**PCT/US2014/067280**

(87) International publication number:
**WO 2015/077751 (28.05.2015 Gazette 2015/21)**

## (54)　**FREESTANDING ULTRA THIN MEMBRANES AND TRANSFER-FREE FABRICATION THEREOF**

FREISTEHENDE ULTRADÜNNE MEMBRANEN UND TRANSFERFREIE HERSTELLUNG DAVON

MEMBRANES ULTRAMINCES AUTO-PORTÉES ET LEUR FABRICATION SANS TRANSFERT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.11.2013 US 201361908695 P**

(43) Date of publication of application:
**05.10.2016 Bulletin 2016/40**

(73) Proprietor: **Northeastern University
Boston, MA 02115 (US)**

(72) Inventors:
- **WADUGE, Pradeep
East Boston, MA 02128 (US)**
- **LARKIN, Joseph
Dorchester, MA 02122 (US)**
- **UPMANYU, Moneesh
Belmont, MA 02478 (US)**
- **KAR, Swastik
Belmont, MA 02478 (US)**
- **WANUNU, Meni
Boston, MA 02132 (US)**

(74) Representative: **Høiberg P/S
Adelgade 12
1304 Copenhagen K (DK)**

(56) References cited:
WO-A1-2012/005857　　WO-A1-2013/188841
US-A1- 2010 069 606　　US-A1- 2010 320 094
US-A1- 2013 240 359　　US-A1- 2013 264 207
US-A1- 2013 264 219　　US-A1- 2013 265 031
US-A1- 2013 309 776　　US-A1- 2013 309 776

- **Pradeep Waduge ET AL: "Programmed Synthesis
of Freestanding Graphene Nanomembrane
Arrays", Small, 18 September 2014 (2014-09-18),
pages 597-603, XP055376278, Germany DOI:
10.1002/smll.201402230 Retrieved from the
Internet:
URL:http://onlinelibrary.wiley.com/store/1
0.1002/smll.201402230/asset/smll201402230.
pdf?v=1&t=j35yevni&s=eb826374288dd545c5e2
2 b5b302612177c06848e [retrieved on
2017-05-26]**
- **GRÉGORY F SCHNEIDER ET AL: "Tailoring the
hydrophobicity of graphene for its use as
nanopores for DNA translocation", NATURE
COMMUNICAT, vol. 4, 15 October 2013
(2013-10-15), pages 1-7, XP008166887, ISSN:
2041-1723, DOI: 10.1038/NCOMMS3619 [retrieved
on 2013-10-15]**

**Description**

CROSS REFERENCE TO RELATED APPLICATIONS

This application claims the benefit of U.S. Provisional Application No. 61/908,695, filed November 25, 2013 and entitled "Production of Transfer-free Freestanding Graphene Membranes".

BACKGROUND

Freestanding ultrathin membranes have attracted a lot of interest in recent years as filtration materials, [1-8] synthetic analogues of biological membranes, [9] and as microelectromechanical sensor (MEMS)-based devices. [10-13] Ultrathin membranes have also found use as substrates for high-resolution nanopores, where the nanopores are tools for single-molecule detection and next-generation DNA sequencing. [14-17] While dielectrics such as silicon nitride (SiN) [18], aluminum oxide [19,20], and silicon oxide [21], have been explored as membrane materials, there is a practical lower limit on membrane thickness for silicon-based membranes (~5-10 nm) [22-24] because silicon oxide and SiN are structurally and chemically more susceptible to damage during [25,26] and after [27] nanopore fabrication. Since high-resolution readout of DNA requires robust ultrathin (<2 nm) membranes, other membrane materials have been explored, including graphene [28-30], boron nitride [31], DNA origami [32-34], hafnium oxide [35,36], and molybdenum disulfide ($MoS_2$) [37]. Due to its atomic thickness, mechanical properties, and unique electrical properties [38-43], graphene in particular allows an additional sensing modality that may allow faster readout speeds and improved resolution. Finally, graphene membranes and their derivatives are impermeable to small molecules and ions, which allows nanopores in such membranes to be tailored for water desalination/purification [44] and selective molecular sieving [3,45,46].

Graphene can be obtained by mechanical exfoliation from graphite flakes [47,48], chemical oxidation/reduction [49], chemical vapor deposition (CVD) [50], and epitaxial growth [51]. In this context, CVD-assisted graphene growth on appropriate catalytic metal surfaces has garnered considerable interest due to its relative ease of synthesis, low cost of production of large-area high-quality graphene, and lack of intense mechanical and chemical treatments. While various approaches for graphene membranes have been reported for nanopore-based DNA analysis [28-30,52], these approaches require graphene transfer to an appropriately perforated substrate. Although considerable progress has been made in graphene transfer techniques [30,53-55], no process offers a scalable approach to produce a large number of membranes for use in nanopore and other membrane-related experiments. In addition, synthesize-then-transfer-graphene protocols [30] for producing graphene can degrade the quality of the membranes by introducing wrinkles, cracks, and contamination during the transfer process. Recently, freestanding graphene membranes have been produced on TEM grids by a transfer-free approach on larger (~30μm) apertures [56], although the ionic permeability of these membranes were not studied.

US2013309776 discloses a nanopore membrane device where a graphene membrane is disposed onto a substrate by transfer using micro-manipulators.

Consequently, there is need for methods of making ultrathin, freestanding graphene membranes. In particular, methods are needed for making intact, ion-impermeable freestanding graphene membranes and methods of introducing small (0.5 - 50 nm) nanopores in such membranes without otherwise affecting membrane integrity. In addition, methods for avoiding membrane transfer would be advantageous.

SUMMARY OF THE INVENTION

Described herein are devices containing freestanding, ultrathin (<10 nm thick) membranes and methods of making such devices. Also described are methods of using devices containing freestanding ultrathin membranes for determining the sequence of a polynucleotide and for desalination of aqueous solutions.

The present invention provides therefore a nanopore membrane device according to independent claim 1, a method of making said device according to independent claim 15 and methods of using such device for determining a sequence of bases of a polynucleotide according to independent claims 16 and 17. Preferred embodiments of the invention are set forth in the dependent claims.

In some embodiments, the membrane is less than about 7.5 nm, less than about 5 nm, less than about 4 nm, less than about 3 nm, less than about 2 nm, or less than about 1 nm thick.

In some embodiments, the passivating material is $HfO_2$ or $TiO_2$. In some embodiments, the layer of passivating material is about 5 nm, about 7.5 nm, about 10 nm, about 15 nm, about 20 nm, or about 40 nm thick.

According to the present invention, the membrane contains one or more nanopores.

In some embodiments, the graphene membrane contains a plurality of nanopores. In some embodiments, each nanopore has a diameter of about equal length. In some embodiments, each nanopore has a diameter of about 0.5 nm, about 0.6 nm, about 0.7 nm, about 0.8 nm, about 0.9 nm, about 1 nm, about 1.5 nm, about 2 nm, about 2.5, about 3 nm, about 4 nm, about 5 nm, about 6 nm, about 7 nm, about 7.5 nm, about 8 nm, about 9 nm, about 10 nm, about 12 nm, about 15 nm, about 20 nm, about 30 nm, about 40 nm, or about 50 nm.

In some embodiments, the substrate contains silicon nitride, aluminum oxide, or hafnium oxide. In some embodiments, the substrate is about 100 nm thick.

In some embodiments, the substrate has multiple wells. In some embodiments, each well has the same diameter. In some embodiments, different wells have different diameters. In some embodiments, the substrate has a set of one or more larger wells and a set of one or more smaller wells. In some embodiments, each well has a diameter of less than 2 $\mu$m, less than 1.5 $\mu$m, less than 1.2 $\mu$m, less than 1 $\mu$m, less than 750 nm, less than 500 nm, less than 400 nm, less than 300 nm, less than 200 nm, or less than 100 nm. In some embodiments, the wells in one set have a diameter of about 800 nm, and the wells in another set have a diameter of about 450 nm.

In some embodiments, the membrane is made of $Bi_2Se_3$, bismuth strontium calcium copper oxide, $Bi_4Ti_3O_{12}$, boron nitride, boron carbon nitride, $(Ca,Sr)_2Nb_3O_{10}$, $Ca_2Ta_2TiO_{10}$, carbon nitride, Cu oxide, $Cu_2O$, CuO, $Cu_2O_3$, $Eu(OH)_2$, fluorographene, GaSe, GaTe, graphene, graphene oxide, InSe, $LaNb_2O_7$, MnO, $MoO_3$, $MoSe_2$, $MoS_2$, MoTe, $Ni(OH)_2$, $NiSe_2$, $NbSe_2$, $NbS_2$, $RuO_2$, $TaO_3$, $TaS_2$, TiO, $TiS_2$, VO, $WO_3$, $WSe_2$, $WS_2$, WTe, ZrSe, or ZrS. In some embodiments, the membrane is made of graphene. In some embodiments, the graphene membrane is less than about 5 atomic layers, less than about 4 atomic layers, less than about 3 atomic layers, or less than about 2 atomic layers thick.

In some embodiments, the graphene at each nanopore has been stabilized to minimize chemical reactivity of the graphene with molecules, ions, and solutes that pass through the nanopore. In some embodiments, that graphene has been stabilized by hydrogenation, hydroxylation, treatment with amphiphilic molecules, biological peptides, or other surface active agents (surfactants).

In some embodiments, the graphene membrane is coated with one or more amphiphilic molecules non-covalently bound to its upper and lower surfaces. In some embodiments, the amphiphilic molecule contains a hydrophobic portion comprising pyrene. In some embodiments, the amphiphilic molecule contains a hydrophilic portion containing an ethylene glycol.

In some embodiments, the device contains a hydrophilic layer attached to the surface of each graphene membrane external to the well and to the lower surface of the substrate. In some embodiments, the hydrophilic layer contains $Al_2O_3$, $SiO_2$, $TiO_2$, or $HfO_2$. In some embodiments, the hydrophilic layer is less than about 30 nm, less than about 20 nm, less than about 15 nm, less than about 10 nm, or less than about 5 nm thick.

In some embodiments, the device has a nanopore in the hydrophilic layer that is aligned with a nanopore in the membrane, e.g., a graphene membrane. In some embodiments, the device has a lipid bilayer non-covalently bound to the hydrophilic layer, the lipid bilayer covering the nanopore in the hydrophilic layer and the graphene membrane. In some embodiments, device contains a biological nanopore situated in the region of the lipid bilayer covering the nanopore.

In some embodiments, the device contains a first lipid monolayer non-covalently bound to the surface of the graphene membrane internal to the well and a second lipid monolayer non-covalently bound to the surface of the graphene membrane external to the well. In some embodiments, the device has one or more nanopores in the graphene membrane to which the lipid monolayers are bound, the lipid monolayers forming a bilayer in the region of the nanopore. In some embodiments, the device contains a biological nanopore disposed in the lipid bilayer.

In some embodiments, the biological nanopore is alpha-hemolysin, MspA porin, or ClyA porin.

In some embodiments, the device contains a supporting structure attached to the upper surface of the substrate, the supporting structure containing a window that provides access to the well. In some embodiments, the device contains an insulating layer attached to the upper surface of the substrate and a supporting structure attached to the insulating layer. In some embodiments, the supporting structure is made of silicon, silicon dioxide, glass, quartz, or mica . In some embodiments, the insulating layer is made of silicon, silicon dioxide, glass, quartz, or mica. In some embodiments, the supporting structure and the insulating layer, if present, contain a plurality of windows, each window providing access to at least one well. In some embodiments the supporting structures contain one or more scored lines between two or more windows, the scored lines enabling the division of the device into two or more pieces, each piece containing one or more windows.

In some embodiments, the device has at least 5, at least 10, at least 20, at least 50, at least 100, at least 150, or at least 200 windows.

In one aspect, the invention includes an apparatus for the study of polynucleotides, the apparatus having: a nanopore membrane device of the invention, wherein the membrane is positioned between a first fluid reservoir and a second fluid reservoir; an electrode pair having a first electrode disposed in the first fluid reservoir and a second electrode disposed in the second fluid reservoir; and a circuit capable of detecting an electrical signal that correlates with the sequence of the polynucleotide.

In one aspect, the invention includes an apparatus for the study of polynucleotides, the apparatus having: a graphene nanopore membrane device of the invention, wherein the graphene membrane is positioned between a first fluid reservoir and a second fluid reservoir; a first electrode pair having a first electrode disposed in the first fluid reservoir and a second electrode disposed in the second fluid reservoir, wherein the first electrode pair is capable of applying an electrical field between the fluid reservoirs across the graphene membrane; a second electrode pair having a first electrode and second electrode on opposite sides of the nanopore, wherein the second electrode pair is capable of applying an electrical field laterally through the graphene membrane and across the nanopore of said device; and a circuit capable of detecting an electrical signal from the second electrode pair that correlates with the sequence of the polynucleotide.

In some embodiments, the apparatus includes a processor for processing signals from the electrodes. In some embodiments, the processor is capable of identifying a polynucleotide, a component of a polynucleotide, or a precursor of a polynucleotide from a signal provided by the electrical sensor.

In some embodiments, the apparatus contains a plurality of devices of the invention. In an example not forming part of the present invention, an apparatus for removal of one or more ionic solutes from an aqueous solution is provided, the apparatus having a plurality of devices containing graphene membranes having a plurality of nanopores.

In another aspect, the invention includes a method of making a membrane device of the invention, the method including the steps of: providing a substrate having an upper surface, a lower surface, and an aperture, the aperture having one or more walls connecting the upper and lower surfaces and forming a well; depositing a passivating layer on the lower surface of the substrate; depositing a sacrificial layer having an upper surface and a lower surface on the passivating layer and across the aperture, thereby forming a floor of the well; forming on the upper surface of the sacrificial layer and/or the passivating layer a membrane that extends across the aperture; and removing the sacrificial layer, leaving the membrane as the floor of the well.

In some embodiments, the sacrificial layer contains Cu, Fe, Ni, Pd, or Pt. In some embodiments, the sacrificial layer contains Cu. In some embodiments, the sacrificial layer is 200 nm thick. In some embodiments, the Cu-containing sacrificial layer is formed by thermal evaporation. In some embodiments, the Cu-containing sacrificial layer is removed by dissolving the Cu in ammonium persulfate.

In some embodiments, the membrane is made of graphene and is made by carbon vapor deposition of methane and hydrogen gases.

In some embodiments, the method includes making a plurality of membrane devices.

In some embodiments, at least 60% , at least 70% , at least 80%, at least 90%, or at least 95% of the membranes produced by the method are intact. In some embodiments, at least 60% , at least 70% , at least 80%, at least 90%, or at least 95% of the membranes produced by the method have a conductance of less than $1 \, nS/\mu m^2$

According to the present invention, the method comprises creating one or more nanopores in the membrane. In some embodiments, the nanopores are created by an electron beam or an ion beam.

In another aspect, the invention includes a method of determining the sequence of bases of a polynucleotide using the apparatus of the invention, the method including the steps of: providing an apparatus that includes (1) a device with a freestanding ultrathin membrane having one or more nanopores positioned between two fluid reservoirs, (2) an electrode pair including one electrode disposed in the first fluid reservoir and a second electrode disposed in the second fluid reservoir, and (3) a circuit capable of detecting an electrical signal; adding an aqueous solution comprising the polynucleotide to the first reservoir and adding an aqueous solution to the second reservoir; applying an electrical field between the first and second electrodes, wherein the electrical field causes one or more charged molecules to transit through the nanopore; measuring a signal due to the transit of the one or more charged molecules through the nanopore; and determining the sequence of bases of the polynucleotide from a previously determined correlation between identities of the bases and the signal.

In some embodiments, the signal is optical. In some embodiments, the signal is electrical. For example, the signal may be a change in current through the nanopore.

In some embodiments, the charged molecules that transit through the nanopore are copes of the polynucleotide whose sequence is being determined. In some embodiments, the polynucleotide that transits through the nanopore is single-stranded. In some embodiments, the polynucleotide that transits through the nanopore is double-stranded. In some embodiments, the polynucleotide that transits through the nanopore contains both single-stranded and double-stranded portions.

In some embodiments, the charged molecules that transit through the nanopore include four synthetic molecules that correspond to each of the four bases that naturally occur in a given polynucleotide, for example, DNA or RNA, wherein each synthetic molecule confers a change in current as it passes through the nanopore, the change in current due to each synthetic molecule being distinct from the change in current due to each of the other three synthetic molecules.

In some embodiments, the first electrode is the anode and the second electrode is the cathode. In some embodiments, the first electrode is the cathode and the second electrode is the anode. In some embodiments, the first electrode alternates between being the anode and the cathode and the second electrode alternates between being the cathode and the anode.

In some embodiments, the polynucleotide transits through the nanopore in a direction determined by an electromotive force provided by the electrical field. In some embodiments, the polynucleotide transits through the nanopore against an electromotive force provided by the electrical field. In some embodiments, the polynucleotide transits through the nanopore alternately with and against an electromotive force provided by the electrical field.

In some embodiments, the force that drives transit of the polynucleotide against the electromotive force is provided by a magnetic field. In some embodiments, the polynucleotide is bound to a magnetic bead. In some embodiments, the force that drives transit of the polynucleotide against the electromotive force is provided by a polynucleotide polymerase.

In some embodiments, the first reservoir contains an enzyme. In some embodiments, the enzyme is DNA polymerase, RNA polymerase, DNA exonuclease, RNA exonuclease, DNA helicase, or RNA helicase. In some embodiments, the enzyme is bound to the membrane adjacent to the nanopore.

In some embodiments, the first reservoir contains an oligonucleotide primer capable of hybridizing with the polynucleotide. In some embodiments, the oligonucleotide primer is bound to the membrane adjacent to the nanopore.

In another aspect, the invention includes a method of determining the sequence of bases of a polynucleotide using the apparatus of the invention, the method including the steps of: providing an apparatus that includes (1) a device with a freestanding ultrathin graphene membrane having one or more nanopores positioned between two fluid reservoirs, (2) a first electrode pair including one electrode disposed in the first fluid reservoir and a second electrode disposed in the second fluid reservoir, and (3) a second electrode pair including electrodes in contact with the graphene membrane on opposite sides of the nanopore, wherein the second electrode pair is capable of applying an electrical field laterally through the graphene membrane, and (4) a circuit capable of detecting an electrical signal from the second electrode pair; adding an aqueous solution comprising the polynucleotide to the first fluid reservoir and adding an aqueous solution to the second fluid reservoir; applying an electrical field between the first pair of electrodes, wherein the electrical field causes one or more charged molecules to transit through the nanopore; applying an electrical field between the second pair of electrodes; measuring a signal between the second pair of electrodes due to the transit of the one or more charged molecules through the nanopore; and determining the sequence of bases of the polynucleotide from a previously determined correlation between identities of the bases and the signal.

In another example that does not form part of the present invention, a method of deionizing an aqueous solution comprising water and unwanted ions is provided, said method including the steps of: providing a device of the invention; applying a pressure to forcibly flow the aqueous solution into the well, thereby causing molecules of water to flow through the nanopores of the membrane and exit the well and causing the unwanted ions to be retained in the well; and collecting the molecules of water that have exited the well.

## BRIEF DESCRIPTION OF THE DRAWINGS

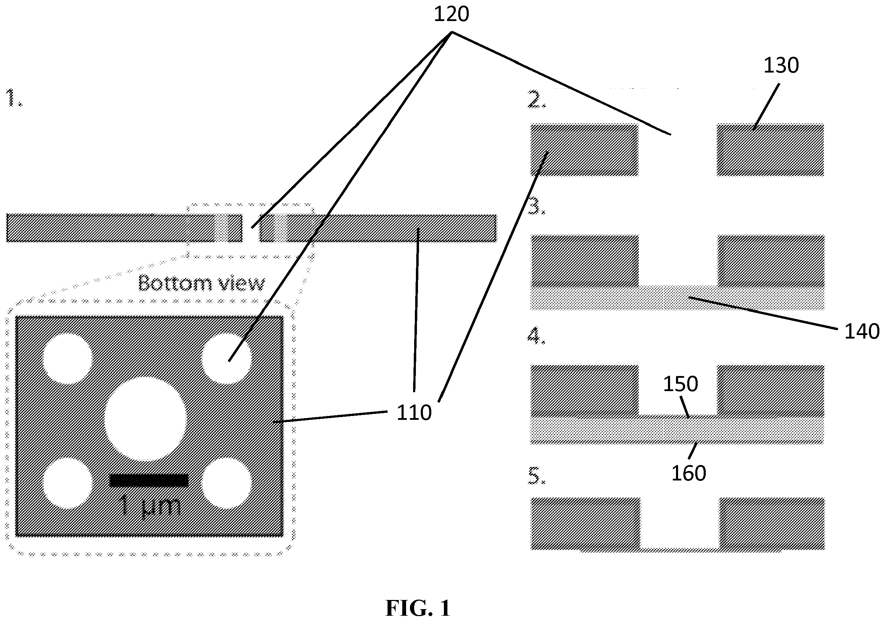

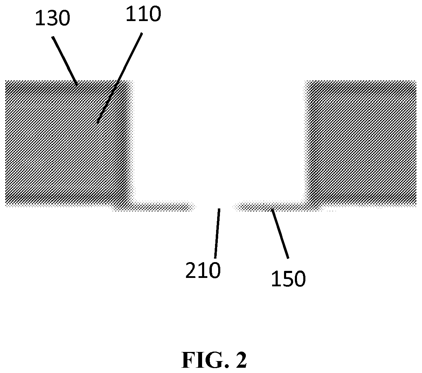

FIG. 1 is a schematic of the fabrication scheme for making a device of the invention. Panel (1) is a schematic of a side view and bottom view of a substrate having five apertures for making wells in a device of the invention. Side views of one of the apertures in panel (1) are shown after (2) deposition of a passivating layer, (3) deposition of a sacrificial layer, (4) formation of upper and lower membranes, and (5) removal of the sacrificial layer and lower membrane. FIG. 2 is a schematic of a well having a nanopore in the membrane.

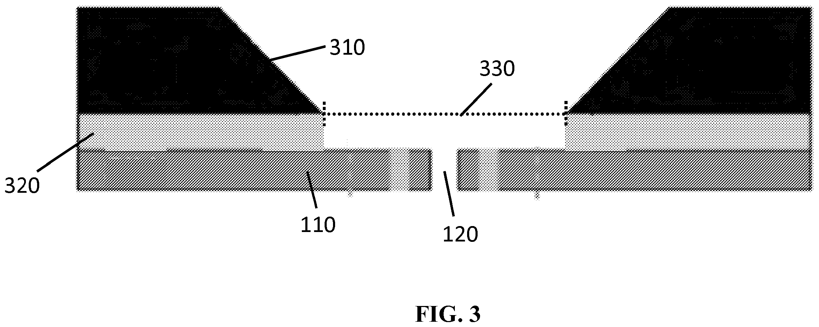

FIG. 3 is a schematic side view of an aperture in the substrate during the fabrication process. The substrate is attached to an insulating layer, which is attached to a support structure. The membrane has not yet been formed at this stage.

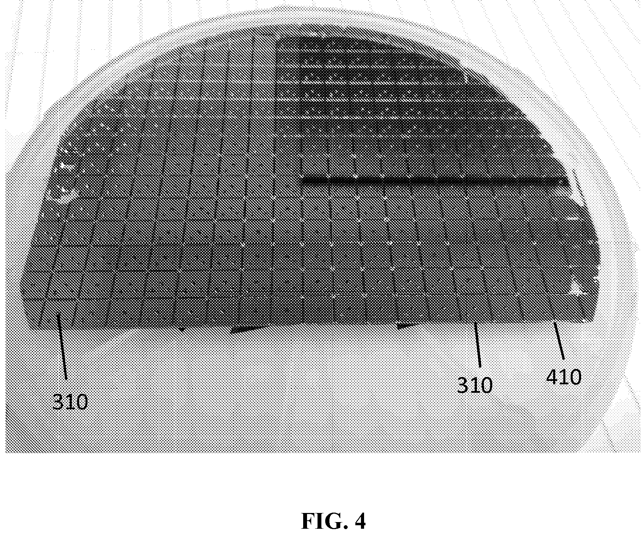

FIG. 4 is an image of a chip having scored lines in the supporting structure, which can be cut to divide the chip into smaller devices for separate use.

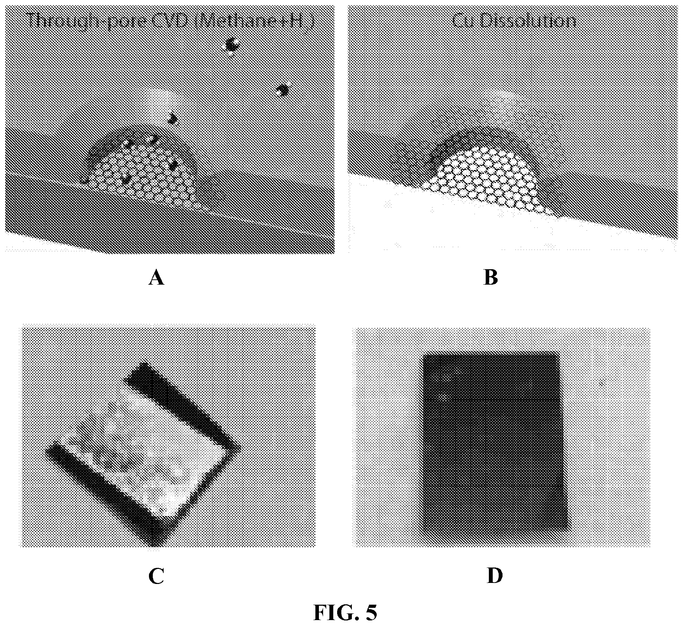

FIG. 5A is a representation of a top view of a well after formation of graphene membranes (step 4 in FIG. 1) by carbon vapor deposition using methane and hydrogen gases in an embodiment of the invention. FIG. 5B is a representation of a top view of a well after removal of the Cu sacrificial layer (step 5 in FIG. 1) by etching of Cu with ammonium persulfate in an embodiment of the invention. FIG. 5C is a bottom view optical image of a device after formation of graphene membranes. Graphene membranes are not visible, Cu sacrificial layer appears light, and supporting structure appears dark. FIG. 5D is a bottom view optical image of a device after removal of the Cu sacrificial layer and bottom graphene.

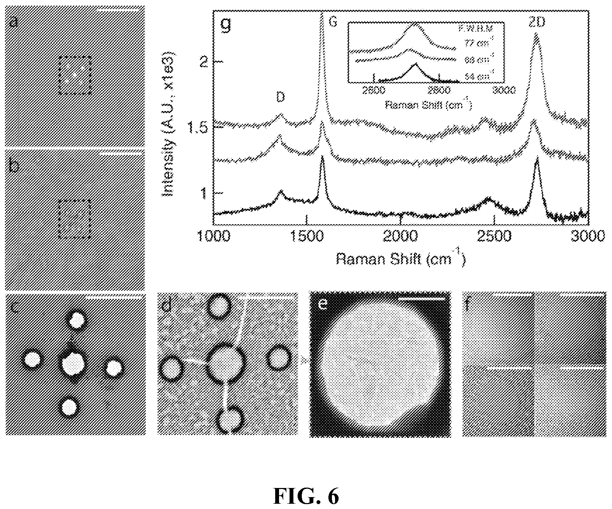

FIG. 6A is a back-illuminated optical image of a low-stress, freestanding SiN substrate with five apertures (dashed box) after deposition of a $HfO_2$ passivating layer but before deposition of the Cu sacrificial layer, i.e., between steps 2 and 3 of FIG. 1 (scale bar = 20 $\mu$m). FIG. 6B is an optical image of the substrate in FIG. 6A after graphene growth and Cu removal, i.e., after step 5 of FIG. 1 (scale bar = 20 $\mu$m). FIG. 6C is bright-field (BF) TEM image of the aperture array after deposition of a $HfO_2$ but before deposition of Cu, i.e., between steps 2 and 3 of FIG. 1 (scale bar = 2 $\mu$m). FIG. 6D is a BF-TEM image of the same substrate shown in FIG. 6C after CVD-assisted graphene growth and Cu removal. FIG. 6E is a TEM image of one of the five apertures of FIG. 6D, revealing graphene nano-domains of varying contrast, i.e., thickness (scale bar = 100 nm). FIG. 6F shows BF-TEM images of freestanding graphene membranes after nanopores have been created in the membranes. Four nanopores are shown (scale bars = 5 nm). FIG. 6G shows the Raman spectra of three different freestanding graphene membranes. These membranes did not have nanopores. Inset shows FWHM values of the 2D-peaks from single-Lorentzian fits (66 $\pm$ 12 cm$^{-1}$), which indicate a multi-layer graphene structure.

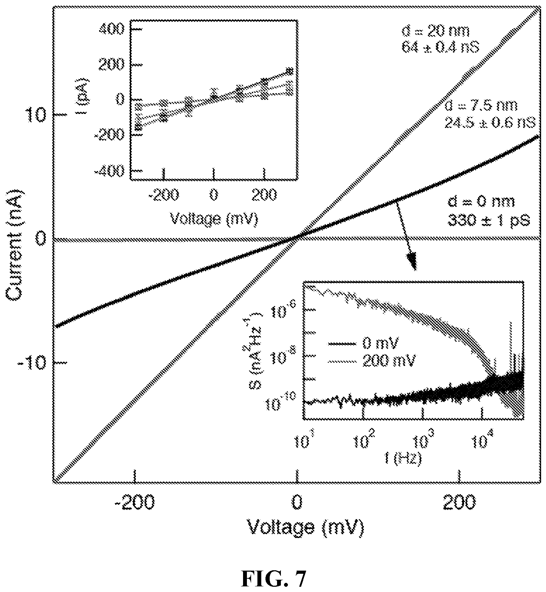

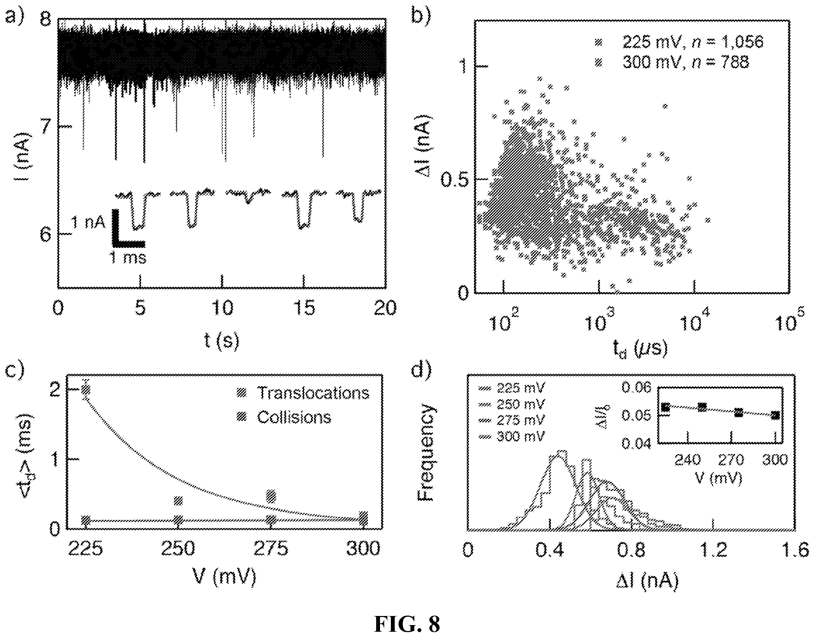

FIG. 7 shows the current-voltage curves of a device having a cluster of 5 wells, with each well having a graphene membrane, as shown in FIG. 6D. The devices had no nanopores in their graphene membranes (red), a ~7.5 nm-diameter nanopore in one of the five graphene membranes (black), and a ~20 nm-diameter nanopore in one of the five graphene membranes (blue). Conductance values for these curves are indicated in the figure. Top inset: current-voltage curves for four different 5-well devices having graphene membranes without nanopores. Bottom inset: noise power spectral densities (PSD) for the device with a ~7.5 nm-diameter nanopore in one of the give graphene membranes at 0 mV and 200 mV applied voltage (sampling rate = 250 kHz, low-pass filtered at 10 kHz, T = 25 °C, 1 M KCl, pH 8.3). FIG. 8A is a graph of continuous ~20-second long current vs. time trace of a 5-well device having a ~7.5 nm-diameter nanopore in one of the five graphene membranes at 300 mV applied voltage after the addition of 8 nM linear 2000 bp double-stranded DNA molecule to the cis chamber. Downward spikes correspond to DNA interactions with the nanopore. Inset shows a magnified view of representative events, extracted from the trace following analysis using OpenNanopore software (fit shown as red curve). FIG. 8B is a scatter plot of $\Delta I$ vs. $t_d$ for a 5-well device with a ~7.5 nm-diameter nanopore in one of the give graphene membranes at 225 mV and 300 mV (n = 1,056 and n = 788, respectively). FIG. 8C shows the mean $t_d$ values as a function of voltage, which correspond to translocation and collision timescales. FIG. 8D shows histograms of $\Delta I$ at different voltages in the range 225-300 mV. Inset shows the fractional current $\Delta I / I_o$, which remains constant in the tested voltage range.

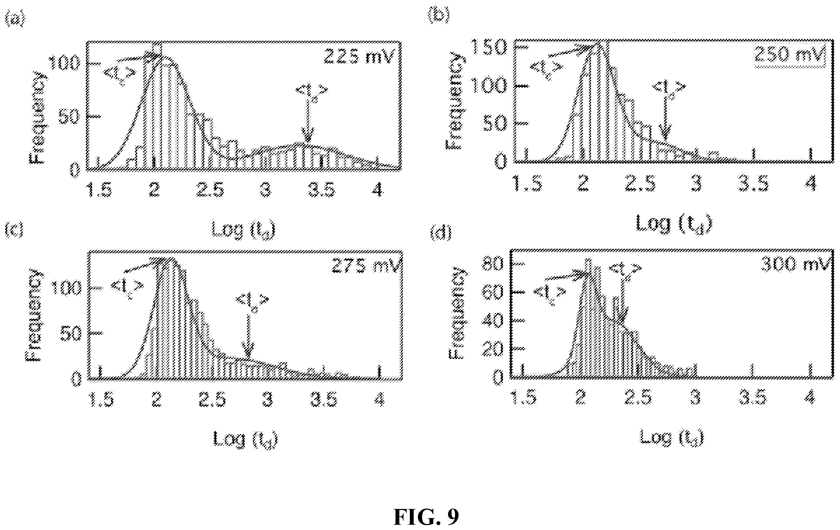

FIG. 9A is a histogram of logarithm of dwell time of double-stranded DNA in a 5-well device with a ~8 nm-diameter nanopore in one of the give graphene membranes at 225 mV bias voltage. The distribution fits to two Gaussians, where the longer dwell time represents the DNA translocations, while the other represents the collisions. FIG. 9B is a histogram as in 8A but using 250 mV bias voltage. FIG. 9C is a histogram as in 8A but using 275 mV bias voltage. FIG. 9D is a histogram as in 8A but using 300 mV bias voltage.

## DETAILED DESCRIPTION OF THE INVENTION

The invention provides freestanding, crack-free ultrathin (< 10 nm thick) membranes. Also provided is a novel approach for in-situ fabrication of large arrays of freestanding ultrathin membranes. Ultrathin graphene membranes are grown directly onto an array of sub-micrometer apertures in a scalable method. The membranes grow precisely above the apertures to yield crack-free membranes, as determined by ionic current measurements. Because the method does not involve transfer of membranes after they have been synthesized, the approach is more practical for obtaining freestanding ultrathin membranes with high yield. The present invention also provides methods of using freestanding ultrathin membranes for determining the sequence of bases in a polynucleotide and for removal of ions from an aqueous solution.

The invention includes a method of making devices containing freestanding, crack-free ultrathin membranes. FIG. 1 shows an embodiment of the process. In this embodiment, the fabrication process starts with a substrate (110) having an upper surface, a lower surface, and one or more apertures (120). Each aperture, which has one or more walls, forms a well. The substrate is then coated with a layer of passivating material (130) on the upper surface, lower surface, and walls of the aperture of the substrate. In alternate embodiments, the passivating layer covers only the lower surface of the substrate, or only the lower surface and walls of the aperture of the substrate. On the passivating layer on the lower surface of the substrate, a sacrificial layer (140) is deposited. The sacrificial layer extends across the aperture of the substrate, creating a floor of the well formed by the aperture. Upper (150) and lower (160) membranes are then formed on the sacrificial layer on the floor of each well. In alternate embodiments of the method, only an upper membrane is formed. Because the membranes are ultrathin, the upper membrane extends beyond the floor of the well to become sandwiched between the sacrificial and passivating layers. The sacrificial layer and lower membrane are then removed. The upper membrane, which is attached at its periphery to the passivating layer, remains attached to the passivating layer, leaving a freestanding ultrathin membrane as the floor of the well.

In examples not being part of the present invention, a passivating layer is not used. In such examples, the periphery of the upper membrane extends beyond the floor of the well to become sandwiched between the sacrificial layer and substrate. When the sacrificial layer and lower membrane, if present, are removed, the upper membrane remains attached to the substrate, leaving a freestanding ultrathin membrane as the floor of the well.

In other examples not being part of the present invention, a sacrificial layer is not used. In such examples, a passivating layer may or may not be used. If a passivating layer is used according to the present invention, the membrane is formed directly on the passivating layer and extends laterally to cover the aperture to create a floor of the well formed by the aperture, thereby forming a freestanding ultrathin membrane. If a passivating layer is not used in said examples, the membrane is formed directly on the lower surface of the substrate and extends laterally to cover the aperture to create a floor of the well formed by the aperture, thereby forming a freestanding ultrathin membrane.

The membrane may be made of any material capable of forming ultrathin membranes impermeable to ionic solutes. For example, the membrane may be made

of $Bi_2Se_3$, bismuth strontium calcium copper oxide, $Bi_4Ti_3O_{12}$, boron nitride, boron carbon nitride, $(Ca,Sr)_2Nb_3O_{10}$, $Ca_2Ta_2TiO_{10}$, carbon nitride, Cu oxide, $Cu_2O$, CuO, $Cu_2O_3$, $Eu(OH)_2$, fluorographene, GaSe, GaTe, graphene, graphene oxide, InSe, $LaNb_2O_7$, MnO, $MoO_3$, $MoSe_2$, $MoS_2$, MoTe, $Ni(OH)_2$, $NiSe_2$, $NbSe_2$, $NbS_2$, $RuO_2$, $TaO_3$, $TaS_2$, TiO, $TiS_2$, VO, $WO_3$, $WSe_2$, $WS_2$, WTe, ZrSe, or ZrS.

The substrate may be made of a low-dielectric material. For example, the substrate may be made of silicon nitride, silicon oxide, aluminum oxide, or hafnium oxide. The substrate may be, for example, about 25 nm, about 50 nm, about 75 nm, about 100 nm, about 200 nm, or about 500 nm thick.

The substrate may contain one well or multiple wells. For example, the substrate may contain at least 2, at least 3, at least 4, at least 5, at least 10, at least 20, at least 40, at least 60, at least 80, at least 100, at least 150, or at least 200 wells. The wells in the substrate may be uniform in size, or the substrate may have sub-populations of wells of different sizes. For example and without limitation, the substrate may have a single larger central well surrounded by four smaller wells.

The wells may take different shapes. For example, the wells may be cylindrical, having a diameter at the floor of the well equal to the diameter of the opening of the well. The wells also may be conical, having a diameter at the floor of the well that is greater than or less than the diameter of the opening of the well. The wells may have both cylindrical and conical regions. The depth of the well may vary, depending on the thickness of the substrate.

The freestanding membranes form the floor of the well, and the well and membrane may vary in size. For example, the freestanding membranes at the floor of the well may have a diameter of about 200 nm, about 300 nm, about 400 nm, about 450 nm, about 500 nm, about 600 nm, about 700 nm, about 800 nm, about 900 nm, about 1 $\mu$m, about 2 $\mu$m, about 4 $\mu$m, about 6 $\mu$m, about 8 $\mu$m, or about 10 $\mu$m.

The passivating material may be any material that prevents the substrate from interfering with formation of the membrane, or improves the ability to form membranes on the substrate material. For example, the passivating material may be $HfO_2$ or $TiO2_2$. The layer of passivating material may about 5 nm, about 7.5 nm, about 10 nm, about 15 nm, about 20 nm, or about 40 nm thick. The passivating layer may be applied by any method known in the art. For example, the passivating layer may be applied by atomic layer deposition.

The sacrificial layer may be made of any material that supports formation of the membrane and can be selectively removed by chemical etching without affecting the membrane integrity. For example, the sacrificial layer may be Cu, Fe, Ni, Pd, or Pt. The sacrificial layer may be made by any suitable method known in the art. For example, the sacrificial layer may be made using sputtering or using a vacuum thermal evaporation system.

The membranes may be formed by any method known in the art. For example, graphene membranes may be formed by chemical vapor deposition (CVD) using methane and hydrogen gases. The membranes may be less than 10 nm, less than 9 nm, less than 8 nm, less than 7 nm, less than 6 nm, 5 nm, less than 4 nm, less than 3 nm, less than 2 nm, or less than 1 nm thick. The membranes may be, for example, less than 5 atomic layers, less than 4 atomic layers, less than 3 atomic layers, or less than 2 atomic layers thick.

The sacrificial layer may be removed by any method that removes the sacrificial layer without affecting the integrity of the upper membrane. For example, a Cu sacrificial layer may be etched using 10% ammonium persulfate.

The method can be used to make intact membranes that are essentially free of defects. Defects, such as cracks and holes, allow ions to pass through the membrane, resulting in increased ionic conductance. Therefore, the presence of an intact membrane can be inferred from low ionic conductance across the membrane. For example, while testing using a 1M solution of KC1 at room temperature, the ionic conductance across the membrane may be less than 0.1 $nS/\mu m^2$, less than 0.3 $nS/\mu m^2$, less than 0.5 $nS/\mu m^2$, less than 0.7 $nS/\mu m^2$, less than 1 $nS/\mu m^2$, less than 1.5 $nS/\mu m^2$, less than 2 $nS/\mu m^2$, less than 5 $nS/\mu m^2$, less than 10 $nS/\mu m^2$, or less than 20 $nS/\mu m^2$.

The method reliably produces intact membranes that are free of cracks. For example, the percentage of intact membranes produced by the method may be greater than 60%, greater than 70%, greater than 80%, greater than 85%, greater than 90%, greater than 95%, greater than 98%, or greater than 99%. Whether a membrane is intact may be determined, for example, from its ionic conductance. Thus, a membrane may considered intact if the ionic conductance across the membrane is less than 0.1 $nS/\mu m^2$, less than 0.3 $nS/\mu m^2$, less than 0.5 $nS/\mu m^2$, less than 0.7 $nS/\mu m^2$, less than 1 $nS/\mu m^2$, less than 1.5 $nS/\mu m^2$, less than 2 $nS/\mu m^2$, less than 5 $nS/\mu m^2$, less than 10 $nS/\mu m^2$, or less than 20 $nS/\mu m^2$. The percentage of intact membranes also can be determined by inspection using electron microscopy, in which cracks are identified by their structure.

As shown in FIG. 2, one or more nanopores (210) are created in the membrane. As used herein, a "nanopore" is a pore having a diameter from about 0.5 nm to about 999 nm. The number of nanopores created per membrane may vary depending on the intended application of the membrane. For example, membranes designed for use in determining the sequence of bases in a polynucleotide may have only a single nanopore per membrane. Alternatively, membranes designed for use in deionization of aqueous solutions may have a plurality of nanopores per membrane.

As used herein, "background conductance" is the conductance of the membrane due to cracks and holes in the membrane prior to the creation of nanopores. The creation of nanopores in a membrane increases its conductance. Therefore, background conductance is the conductance of a membrane before nanopores have been created in the membrane.

Pores in the membrane may be made by any method that allows the nanopores size to be precisely controlled and that does not damage the remaining portions of the membrane. For example, electron beams or ion beams can be used to create nanopores in the membrane.

The shape and size of the nanopores in the membrane will vary depending on the application of the membrane. For some applications, the nanopores are circular or nearly circular. For some applications, the circular or nearly circular nanopores have a diameter of about 0.5 nm, about 0.6 nm, about 0.7 nm, about 0.8 nm, about 0.9 nm, about 1 nm, about 1.5 nm, about 2 nm, about 3 nm, about 4 nm, about 5 nm, about 6 nm, about 7 nm, about 7.5 nm, about 8 nm, about 10 nm, about 12 nm, about 15 nm, about 20 nm, about 25 nm, about 30 nm, about 35 nm, about 40 nm, about 45 nm, or about 50 nm. In various embodiments, nanopores may have a size in the range from about 0.5 nm to about 3 nm, from about 0.5 nm to about 5 nm, from about 1 nm to about 10 nm, or from about 0.5 nm to about 50 nm. In membranes that have multiple nanopores, the nanopores may be uniform or nearly uniform in size and shape. Alternatively, in membranes that have multiple nanopores, the nanopores may vary in size and/or shape.

The carbon atoms at the edge of the nanopores in graphene membranes may be passivated to strengthen or stabilize the nanopore or to change its chemical properties. For example, the edges of the nanopores, particularly with graphene membranes, may hydrogenated, hydroxylated, or treated with a functionalized peptide or surfactant. Hydrogenation of carbon atoms at nanopore edges can be used to create more hydrophobic nanopores, and hydroxylation of carbon atoms or treatment with a surfactant at nanopore edges can be used to create more hydrophilic nanopores.

In membranes that have multiple nanopores, the nanopores may be regularly or randomly spaced. For example, the average nanopore spacing may be, for example, about 10 nm, about 20 nm, about 50 nm, about 100 nm, about 200 nm, about 400 nm, about 600 nm, about 1 μm, about 2 μm, or about 3 μm apart.

For some applications, it may be necessary to coat the membranes with other materials to prevent analytes from binding to the membrane. For example, DNA may stick to graphene due to hydrophobic interactions between graphene and the nucleotide bases. Thus, graphene membranes may be coated with other materials to prevent unwanted interactions with the membranes, such as materials that decrease the hydrophobicity and increase the hydrophilicity of the membranes. Graphene membranes may be coated by non-covalent binding of amphiphilic polymers to the membrane surfaces. A two-step method for coating graphene membranes has been described by Schneider et al. [68] In the first step, aminopyrene is adsorbed onto graphene. In the second step, tetraethyleneglycol monomethyl ether N-hydroxysuccinimide ester is added, causing the formation of stable peptide bond between the amine group of aminopyrene and the carbonyl group of N-hydroxysuccinimide ester. Other methods that achieve comparable effects may also be used. Alternatively, graphene membranes may be coated with hydrophilic metallic compounds. For example, coating of graphene membranes with $Al_2O_3$ has been described by Connelly et al. [69] $TiO_2$, $SiO_2$, and $HfO_2$ can also be used. The amphiphilic or hydrophilic coating may be less than 20 nm, less than 15 nm, less than 10 nm, or less than 5 nm thick. The amphiphilic or hydrophilic coating may be applied to a membrane before or after nanopores have been created in the membrane. Consequently, it may be necessary to create nanopores in the amphiphilic or hydrophilic coating as well as the membrane so that the nanopore extends through both the coating and the membrane.

The membranes may also be coated with lipid layers, for example, lipid monolayers and lipid bilayers. The lipid bilayer may interact with the membrane directly or indirectly. For example, the membrane can be coated on one side with a hydrophilic layer, e.g., $Al_2O_3$, and the polar head groups of one layer of the lipid bilayer can bind to the hydrophilic layer. Because the lipid bilayer forms a continuous sheet, the nanopores in the membrane and hydrophilic layer remain covered with a region of freestanding lipid bilayer. Alternatively, each side of a hydrophobic membrane, e.g., graphene, may bind directly to the hydrophobic acyl chains of a lipid monolayer. Consequently, the regions of intact membrane form a three-layered structure in which the membrane is sandwiched between two lipid monolayers. Over the nanopores, however, the acyl chains of one monolayer interact directly the acyl chain from the other monolayer, resulting in a freestanding lipid bilayer over the nanopore. In both of these arrangement, the freestanding lipid bilayers over the nanopores prevent ionic solutes from passing through the nanopores.

For some applications, biological nanopores are integrated into the regions of freestanding lipid bilayer over the nanopores of a membrane to allow passage of charged molecules across the membrane. The biological nanopores may be proteins, for example, nanopore proteins or ion channels. The nanopore proteins may be proteins useful for sequencing polynucleotides. For example, the nanopore protein may be alpha-hemolysin, MspA porin, ClyA porin, or another nanopore protein.

The substrate is attached to a supporting structure and, optionally, an insulating layer. As shown in FIG. 3, in one embodiment the substrate (110) contacts the insulating layer (320), which contacts the supporting structure (310). Because the substrate is thin and thus susceptible to being damaged during handling, the supporting structure provides structural strength and rigidity to preserve the integrity of the well and membrane. The supporting structure may be made of any material suitable for this purpose. For example, the supporting structure may be made of silicon, glass, quartz, sapphire, or mica. An insulating layer may be necessary to electrically insulate the membrane from the supporting structure, so the insulating layer may be made of any material suitable for this purpose. For example, the insulating layer may be made of $SiO_2$ or $Al_2O_3$. The insulating layer may be, for example, 0.5 $\mu$m, 1 $\mu$m, 1.5 $\mu$m, 2 $\mu$m, 2.5 $\mu$m, 3 $\mu$m, 4 $\mu$m, 5 $\mu$m, 6 $\mu$m, 7 $\mu$m, 8 $\mu$m, 9 $\mu$m, or 10 $\mu$m thick. To provide access to the wells, the supporting structure and insulating layer, if present, contain one or more windows (330) in which the substrate is not in contact with the supporting structure or insulating layer. The window in the supporting structure and insulating layer, if present, is at least as large as the well but may be larger. The window may have a uniform diameter across the depth of the window, or the diameter may increase at increasing distance (depth) from the substrate. For example, the window in the supporting structure and insulating layer, if present, may have a diameter of, for example, 1 $\mu$m, 2 $\mu$m, 5 $\mu$m, 10 $\mu$m, 20 $\mu$m, 40 $\mu$m, 80 $\mu$m, 120 $\mu$m, 160 $\mu$m, or 200 $\mu$m at the site of contact between the window and the substrate.

The method of making devices is scalable so that multiple wells and membranes can be made on a single supporting structure, i.e., the devices can be manufactured as "chips." One chip may have, for example, 1, 2, 3, 4, 5, 10, 20, 40, 60, 80, 100, 150, 200, 400, 600, 800, 1000, 2000, 4000, 6000, 8000, 10,000 or more windows, each window containing one or more wells. A chip with multiple nanopores may be designed for use as such, or it may be designed to be divided into smaller devices, each containing one or more windows that contain one or more wells, for separate use, as shown in FIG. 4. Consequently, the supporting structure may have scored lines (410), or structural weak points, in between windows or sets of windows that allow the chip to be readily cut and divided into smaller chips without damaging the ultrathin membranes.

The invention includes composite devices that can be used to determine the base sequence of a polynucleotide. In addition to the substrate and membranes having one or more nanopores as described above, such composite devices may include one or more of the following: a pair a reservoirs capable of holding conductive fluid, the two reservoirs in contact with, but on opposite sides of, the membrane; a first pair of electrodes capable of applying a an electrical field between the two fluid reservoirs, each of the two electrodes in electrical contact with one reservoir; an electrical sensor capable of detecting an electrical signal between the two reservoirs; an electrical sensor capable of detecting a lateral electrical signal in the plane of the membrane; and a processor attached to the electrical sensor, the processor capable of identifying a polynucleotide, a component of a polynucleotide, or a precursor of a polynucleotide from a signal provided by the electrical sensor.

The invention also encompasses methods of using the devices described herein to determine the sequence of bases in a polynucleotide. Several methods for determining the sequence of a polynucleotide using nano-scale nanopores in other materials have been have been described previously, and these methods can be adapted for use with the devices containing freestanding ultrathin membranes described herein. See, for example, WO 2013/185137; US patent no. 8652779; US 2013/0264207; WO 2012/088339; US 2007/0190542; WO 2013/119784; and US 2013/0256118. The methods involve an apparatus having the following: a device with a freestanding membrane having one or more nanopores positioned between two fluid reservoirs; and an electrode pair including one electrode in electrical contact with the one fluid reservoir and another electrode in contact with the other fluid reservoir, the electrodes capable of applying an electrical field; and a circuit capable of detecting an electrical signal. The methods involve the following steps: adding an aqueous solution containing the polynucleotide to one reservoir and adding an aqueous solution to the other reservoir; applying an electrical field between the two electrodes, causing one or more charged molecules to transit through the nanopore; measuring a signal due to the transit of the one or more charged molecules through the nanopore; and determining the sequence of bases of the polynucleotide from a previously determined correlation between identities of the bases and the signal.

The signal measured may be optical. See, for example, US 2013/0256118. Alternatively, the signal measured by be electrical. See, for example, WO 2013/185137; US patent no. 8652779; US 2013/0264207; WO 2012/088339; US 2007/0190542; WO 2013/119784.

The electrode in contact with the reservoir that initially contains the polynucleotide may function as the anode or the cathode, or it may alternate between functioning as the anode and cathode.

The charged molecules that transit through the nanopore may be the polynucleotides. See, for example, WO 2013/185137; WO 2012/088339; US 2007/0190542; WO 2013/119784; and US 2013/0256118. The polynucleotide that passes through the nanopore may be single-stranded, double-stranded, or have a combination of single-stranded and double-stranded portions. See, for example, WO 2013/185137; WO 2012/088339; US 2007/0190542; WO 2013/119784; and US 2013/0256118. Alternatively, the charged molecules that transit through the nanopore may be synthetic molecules that correspond to the four naturally-occurring bases of the polynucleotide. For example, they may be derived from dNTP analogs in which each of the four naturally-occurring nucleotides has been modified by the addition of a synthetic tag via three or more phosphates. See, for example, US patent no. 8652779; US 2013/0264207.

Such analogs are used for synthesis of a new polynucleotide strand by a polymerase situated near the nanopore. In some methods, the synthetic molecule includes a linker and a current blockade label. See US Patent No. 8652779. While dNTP base is paired with its complement on the template strand, the blockade label becomes temporarily lodged within the nanopore. See US Patent No. 8652779. Cleavage of the phosphate linkage by the polymerase allows charged molecule composed of the linker and blockade label to pass through the nanopore. Alternatively, in some methods, the synthetic molecule includes a tag that is unable to enter the nanopore while the dNTP base is paired with its complement on the template strand. See US 2013/0264207. Cleavage of the phosphate linkage by the polymerase allows the charged molecule composed of the tag and one or more phosphates to pass through the nanopore.

The polynucleotides that transit through the nanopores may have modified bases. See, for example, WO 2013/185137. The polynucleotide may have modified bases on only one strand. In some methods, only one strand of the polynucleotide passes through the nanopore, and the strand passing through the nanopore may be the strand with modified bases or the strand without modified bases. If the strand with the modified bases passes through the nanopore, the modified bases may block or alter the flow of ions through the nanopore and thus be identified by measuring the current through the nanopore. Alternatively, the modified bases may alter the kinetics of transit of the polynucleotide through the nanopore. See, for example, WO 2013/185137. For example, transit of a strand of a polynucleotide may be coupled with an enzymatic reaction, such as the synthesis of a new strand of DNA or RNA, the exonucleolytic degradation of a strand or DNA or RNA, or the separation of two strands of DNA or RNA, and the modified base may slow the rate of the enzymatic reaction. See, for example, WO 2013/185137.

The polynucleotides that transit through the nanopores may move with the electromotive force or against it, or they may alternate between moving with and against the electromotive force. The force moving the polynucleotides through the nanopore against the electromotive force may be magnetic. For example, the polynucleotide may be covalently bound to a magnetic bead. See, for example, WO 2013/119784. Alternatively, the polynucleotide may move through a nanopore against an electromotive force due to the pulling forces of an enzyme, for example, a polymerase. See, for example, WO 2013/185137.

The reservoir that initially contains the polynucleotide may also contain an enzyme. For example, it may contain a DNA polymerase, RNA polymerase, DNA exonuclease, RNA exonuclease, DNA helicase, and RNA helicase. See, for example, WO 2013/185137. The enzyme may be bound to the membrane located precisely above or directly adjacent to the nanopore. See, for example, WO 2013/185137; and US patent no. 8652779.

The reservoir that initially contains the polynucleotide may also contain a primer that is capable of hybridizing with the polynucleotide. The primer is a short, usually chemically synthesized oligonucleotide, of appropriate length, for example about 18-24 bases, sufficient to hybridize to a target DNA (e.g. a single stranded DNA) and permit the addition of a nucleotide residue thereto, or oligonucleotide or polynucleotide synthesis therefrom, under suitable conditions well-known in the art. In an embodiment the primer is a DNA primer, i.e. a primer consisting of, or largely consisting of, deoxyribonucleotide residues. The primers are designed to have a sequence which is the reverse complement of a region of template/target DNA to which the primer hybridizes. The addition of a nucleotide residue to the 3' end of a primer by formation of a phosphodiester bond results in a DNA extension product. The addition of a nucleotide residue to the 3' end of the DNA extension product by formation of a phosphodiester bond results in a further DNA extension product. The primer may be bound to the membrane adjacent to the nanopore. See, for example, US 2013/0264207.

EXAMPLES

Example 1: Materials and Methods

Substrates for nanopore fabrication were 5 mm × 5 mm Si chips with a 100-nm-thick SiN film deposited on a 2.5-$\mu$m-thick thermal SiO2 layer, which helps to reduce electrical noise. SiN was protected with a 950 PMMA etch mask, and a small region (2 $\mu$m × 2 $\mu$m square region with a pattern of four 450 nm-diameter holes and a central 800 nm hole) was exposed using Nabity NPGS e-beam writing software on a Hitachi S-4800 scanning electron microscope. Exposed PMMA was developed with 3:1 isopropyl alcohol and methyl isobutylketone, and AFM- and ellipsometry-calibrated thicknesses of SiN were etched in a Technics Micro-RIE Series 800 etcher using sulfur hexafluoride ($SF_6$) at 300 mTorr and 150 W. PMMA was removed using 30 min treatment with acetone and chips were cleaned with hot piranha followed by warm water to remove the residual PMMA. $HfO_2$ films were deposited at 150 °C using a benchtop ALD system (Arradiance Gemstar), with tetrakis(ethylmethylamino) hafnium and $H_2O$ used as a precursor and oxidizer, respectively. A calibrated thickness of Cu was evaporated on the membrane side of the chip using a vacuum thermal evaporation system. Graphene membranes were synthesized using a low-pressure CVD technique in a split tube furnace with a 35 mm O.D. quartz tube as follows: Following Cu deposition, the chips were placed in the center of the furnace and the vacuum system was turned on. Once the pressure inside the furnace became ~11 mTorr, 35 sccm of $H_2$ were allowed to flow and the tube was heated to growth temperature of 1000 °C. Upon reaching 1000 °C the $H_2$ flow rate was reduced to 2 sccm, and 35 sccm of $CH_4$ gas were flowed for 3 h for graphene growth. During the growth process, the vacuum of the whole system was kept under 1.5 Torr. Following deposition, the furnace was allowed to naturally cool down to room temperature under a 35 sccm flow of $H_2$. After removal of the chips from the furnace the Cu was etched using 10% ammonium persulfate, and then the chips were rinsed with DI water and isopropyl alcohol and dried under a gentle flow of dry $N_2$. Raman spectroscopy was carried out using a Jobin Yvon LabRam HR800 spectrometer attached to an Olympus BH2 microscope. Graphene nanopores were fabricated and imaged at Northeastern University using a JEOL 2010FEG transmission electron microscope operating in bright-field mode at 200 kV.

Example 2: Graphene Nanomembrane Fabrication

A process of membrane fabrication is shown in FIG. 1 and FIGS. 5A-5D. First, an array of 5 mm × 5 mm silicon chips, each containing a freestanding low-stress SiN window (~40-80 $\mu$m), was cleaned in hot piranha and then rinsed copiously in warm de-ionized (DI) water, and then dried with a gentle flow of nitrogen ($N_2$) gas. Next, positive electron-beam resist was spun on the chips, and a 2 $\mu$m × 2 $\mu$m portion of the SiN window was irradiated using e-beam lithography such that a pattern of five sub-micron holes was written and subsequently developed. Sub-micrometer holes through the nitride membrane were then generated by controlled etching using an $SF_6$ reactive ion etch (RIE) plasma. Resist was then stripped using acetone and a hot piranha treatment (Step 1). The chips were then placed in an atomic layer deposition (ALD) instrument (Arradiance Gemstar) and a 10 nm thick $HfO_2$ film was deposited on both sides of the chip to passivate the SiN membrane (Step 2). This step was necessary as we found that subsequent graphene growth on unpassivated substrates resulted in contamination with silicon-based crystallites during the CVD process. After passivation, a ~200-nm-thick Cu film was deposited on the bottom of the membrane using thermal evaporation (Step 3 and FIG. 5C). Graphene was then directly grown onto the Cu film over nanoapertures on SiN window using CVD at 1000 °C using $CH_4$ and $H_2$ gases (Step 4 and FIG. 5A). Following CVD the Cu catalyst was dissolved using 10% ammonium persulfate, and the device was rinsed with DI water and isopropanol (Step 5 and FIGS. 5B and 5D). Finally, nanopores were drilled through the graphene membranes using the electron beam of a transmission electron microscope.

Example 3: Graphene Nanomembrane Characterization

FIG. 6A shows a back-illuminated optical microscopy image of a low-stress freestanding SiN membrane with five nanoholes fabricated using e-beam lithography. Deposition of Cu on the membrane results in a layer of Cu catalyst on one side of the hole array. FIG. 6B shows a back-illuminated optical image of the same membrane after 3-hour CVD graphene growth, following Cu dissolution. While the holes appear to be transparent, they are covered with graphene; this is illustrated by comparative TEM images before (FIG. 6C) and after (FIGS. 6D and 6E) CVD-assisted graphene growth. In FIG. 6C, which shows the nano-holes passivated with a thin film of $HfO_2$, holes are clearly present. The black rings observed around the nano-holes are due to a high contrast from the $HfO_2$ layer inside the holes. However, following graphene growth the nano-holes are all covered with freestanding graphene membranes. In FIG. 6E, it is clear that some unetched nanoscopic Cu domains remain even after thorough dissolution treatment using ammonium persulfate, probably due to its trapping between different graphene layers. The contrasting patches seen in FIG. 6E correspond to thickness variations in the CVD-grown graphene as the graphene flakes themselves are comprised of different number of graphene layers localized on the membrane. The few-layer graphene flakes are thin enough for rapid (5-10 seconds) nanopore fabrication using a highly focused electron beam of a TEM, noticeably faster than the typical time for drilling similar nanopores in conventional SiN membranes using similar conditions. [57] Several nanopores in the diameter range of 3-9 nm fabricated in different graphene devices are shown in FIG. 6F. Raman spectra, which are typically used to characterize the thickness and quality of graphene, [58,59] are shown in FIG. 6G. The three curves shown represent spectra collected from the area of the nano-hole arrays in three different devices (though the laser beam could not be restricted to solely within the hole area). Pronounced 2D, G, and a weaker D band, are observed at ~2720, -1582, and ~1360 cm -1, respectively. It is well known that the quality and uniformity of CVD graphene is characterized by the $I_{2D}/I_G$ ratio, the peak positions of the 2D and G bands, and the full-width at half-maximum (FWHM) of the 2D band based on single-Lorentzian least squares fits. [50,60,61] In particular, the $I_{2D}/I_G$ ratio decreases as the number of graphene layers increases. Peak positions of G and 2D bands, the $I_{2D}/I_G$ ratio and FWHMs of 2D bands of the Raman spectra of three devices that tested here confirm the formation of multilayer graphene (see Example 7). [62] The presence of a weak D-band with CVD-assisted grown graphene is expected because the laser spot (~1 $\mu$m) is much larger than the size of a typical graphene domain (20-100 nm).

Example 4: Ionic Conductance Measurements

The ionic conductance of transfer-free freestanding graphene membranes were studied by mounting graphene nano-membrane devices into a custom-made CTFE holder that allows 1 M KC1 electrolyte solution to be placed on either side of the membrane. Ag/AgCl electrodes immersed in each electrolyte bath were used to apply voltage in the range of ±300 mV across the membrane, and ion currents were measured using an Axopatch 200B patch-clamp amplifier. A current-voltage curve for a typical graphene nano-membrane device without a nanopore (red curve), as well as for a 7.5 nm (black curve) and a 20 nm (blue curve) diameter nanopores are shown in FIG. 7. First, the mean conductance of bare graphene nanomembranes was in the range of 100-500 pS, as measured from the slopes of the current-voltage curves for ten separate devices (four of them shown in inset to FIG. 7). Such low conductance values, on par with monolayer graphene membranes, [28] corresponds to an extremely low pinhole fraction in the membrane. Of 50 devices tested over the course of the project, only 20% exhibited leakage that would correspond to unintentional holes or tears. In contrast to the pristine devices, the ion conductance of the 7.5 nm and 20 nm diameter nanopores is much greater due to ion transport through the nanopore orifice. For an infinitely thin insulating membrane with a circular nanopore of diameter d, the ionic conductance G through the nanopore is defined as: [63]

$$G = \sigma\, d \quad (1)$$

where $\sigma$ = 0.096 S/cm is the measured specific conductance of buffer at 25°C. The calculated G values for transfer-free graphene membranes from known $\sigma$ and d, indicated in FIG. 7, yield d values of 2.5 nm and 6.7 nm for the 7.5 and 20 nm nanopores, respectively. This 3-fold discrepancy between TEM based and measured diameters becomes clear in the investigation of DNA translocation through these nanopores in the next section.

Example 5: Noise Characteristics

The noise characteristics of ion current signals through nanopores have been studied profusely. Uncoated graphene nanopores exhibit a high noise as compared with silicon nitride and other hydrophilic nanopores in inorganic dielectrics. The inset to FIG. 7 presents noise power spectral density (PSD) plots for a ~7.5 nm diameter graphene nanopore at 0 mV and 200 mV. Despite a similar PSD for the transfer-free graphene nano-membrane to that of a transferred graphene membrane with a similar diameter nanopore, [29] the 1/f noise increases drastically and dominates the noise upon application of a 200 mV bias. This increase in 1/f noise is likely due to the poor surface characteristics of the unmodified graphene surface, i.e., hydrophobic patches [64] and mechanically unstable nanopore edges. While coating the nanopores, i.e., with an ALD film of TiO2 [29] or $Al_2O_3$, [65] improves the noise drastically, the properties of the bare untreated graphene surface are reported here. Finally, in all reported measurements, the ~20% of devices which showed a complete current overload (>1000 nS), possibly due to a crack through the membrane during fabrication or handling during device assembly, were not included.

Example 6: Double-Stranded DNA Transport

FIG. 8A shows a characteristic continuous 20-second raw current trace of a 7.5 nm nanopore at 300 mV voltage upon the addition of linear 2000 bp dsDNA to a final concentration of 8 nM into the negatively biased cis chamber. The stochastic appearance of downward spikes corresponds to interactions with individual DNA molecules with the nanopore. Analysis of the pulse characteristics was carried out using OpenNanopore open-source software developed by the Radenovic group. [66] The program uses a cumulative sum algorithm to identify and measure the dwell time ($t_d$) and current blockade amplitude ($\Delta I$) for each spike. The inset to FIG. 8A shows example pulses retrieved from the analysis.

Scatter plots of $\Delta I$ vs. $t_d$ for 225 mV and 300 mV are shown in FIG. 8B. The plot shows a distribution of dwell times for 225 mV that is much broader and longer than observed for DNA transport through similar-diameter nanopores in silicon nitride, for example. [67] In comparison with other graphene nanopores, the mean $t_d$ value observed for 2 kbp dsDNA through the 7.5 nm-diameter graphene nanopore at 225 mV translates to mean velocities that are ~3 times slower than observed for transport of 15 kbp dsDNA through a nanopore in transferred-few-layer graphene at 100 mV. [29] A closer inspection of the dwell time distributions for the experiments reveals two distinct populations that are resolvable in terms of dwell times, which are likely due to brief DNA collisions and full DNA translocations through the nanopore. The broad nature of the translocation population at 225 mV, which ranges from 0.5-10 ms, is possibly a result of sticky interactions of the DNA with the nanopore due to the hydrophobic surface characteristics of graphene. Increasing the voltage to 300 mV increases the force applied to the DNA molecules, which shifts the translocation time distribution to faster dwell times in range 80-500 $\mu$s while greatly reducing the scatter.

Analysis of the most probable dwell time populations ($t_d$) at four voltages in the range 225-300 mV is shown in FIG. 8C (see Example 7 and FIG. 9 for details). Peak $t_d$ values decrease exponentially with increasing voltage, while collision times are independent of the applied bias voltage in the range 225-300 mV. The regular decrease in the spread of $t_d$ values, along with the exponential decrease in peak $t_d$ values with increasing voltage, points to a transition from diffusion-dominated transport at low bias values to drift-dominated transport at higher bias values, and suggests that interactions of DNA with bare graphene significantly impact its transport behavior.

Finally, histograms of $\Delta I$ at each DNA translocation experimental voltage are shown in FIG. 8D. Current blockades increase linearly with voltage in the range 225-300 mV, while the fractional blockade values, i.e., $<\Delta I>/ I_o$, are constant in this range. This suggests that the nanopore size does not change during the course of the experiment and/or at different voltages. Based on the

open nanopore current ($I_o$) values and mean $\Delta I$ values, calculation of the effective nanopore diameter d and effective thickness $h_{eff}$ of the graphene nanopores yields d = 6.5 nm and $h_{eff}$ = 8 nm. As indicated earlier, in the limit of infinitely thin nanopores, the calculated G values based on TEM measurements deviate by three-fold from measured values of G. Using the information from DNA translocation experiments, it was established that the graphene nanopores are indeed thicker than the infinitely thin limit (i.e., single-layer graphene), hence resolving this discrepancy between calculated and experimental d values. This relatively thick value is most likely due to the growth conditions, which can be optimized in future experiments by fine-tuning the graphene growth parameters.

Example 7: Determination of mean DNA dwell times

Mean DNA dwell times, as shown in FIG. 9, were determined as follows. First, a histogram of logarithm of dwell time ($t_d$) for a given data set is plotted to determine the mean peak dwell time ($<t_d>$). Then a least squares fit of the histogram to a Gaussian is performed. The $<t_d>$ is determined from the position of this Gaussian's mean, which is considered as the characteristic dwell time for the experiment. Each distribution has two peaks in the voltage range 225-300 mV - the longer dwell time represents the DNA translocation while the other represents the collisions, which exhibit negligible voltage dependence. As the bias voltage is increased the dwell time peak moves to the left, while the collision peak remains the same. In all cases, the double-stranded DNA (dsDNA) and the nanopore are the same, except the different bias voltages.

REFERENCES

[1] V. Berry, Carbon 2013, 62, 1 - 10.

[2] H. W. Kim, H. W. Yoon, S.-M. Yoon, B. M. Yoo, B. K. Ahn, Y. H. Cho, H. J. Shin, H. Yang, U. Paik, S. Kwon, Science 2013, 342 (6154), 91 - 95.

[3] R. Nair, H. Wu, P. Jayaram, I. Grigorieva, A. Geim, Science 2012, 335 (6067), 442 - 444.

[4] A. W. Hauser, P. Schwerdtfeger, J. Phys. Chem. Lett. 2012, 3 (2), 209 - 213.

[5] L. W. Drahushuk, M. S. Strano, Langmuir 2012, 28 (48), 16671 - 16678.

[6] D.-E. Jiang, V. R. Cooper, S. Dai, Nano Lett. 2009, 9 (12), 4019 - 4024.

[7] S. C. O'Hern, C. A. Stewart, M. S. Boutilier, J.-C. Idrobo, S. Bhaviripudi, S. K. Das, J. Kong, T. Laoui, M. Atieh, R. Karnik, ACS Nano 2012, 6 (11), 10130 - 10138.

[8] H. Du, J. Li, J. Zhang, G. Su, X. Li, Y. Zhao, J. Phys. Chem. C 2011, 115 (47), 23261 - 23266.

[9] L. S. Connelly, B. Meckes, J. Larkin, A. L. Gillman, M. Wanunu, R. Lal, ACS App. Mat. Int. 2014, 6 (7), 5290 - 5296.

[10] A. D. Smith, F. Niklaus, A. Paussa, S. Vaziri, A. C. Fischer, M. Sterner, F. Forsberg, A. Delin, D. Esseni, P. Palestri, Nano Lett. 2013, 13 (7), 3237 - 3242.

[11] M. Liao, Y. Koide, Critical Rev. Solid State Mater. Sci. 2011, 36 (2), 66 - 101.

[12] S. Basu, P. Bhattacharyya, Sens. Actuat. B: Chem. 2012, 173, 1 - 21.

[13] T. Miao, S. Yeom, P. Wang, B. Standley, M. Bockrath, Nano Lett. 2014, DOI: 10.1021/nl403936a.

[14] K. K. Saha, M. Drndic, B. K. Nikolic, Nano Lett. 2012, 12 (1), 50 - 55.

[15] M. Puster, J. A. Rodriguez-Manzo, A. Balan, M. Drndic, ACS Nano 2013, 7 (12), 11283 - 11289.

[16] J. Wilson, M. Di Ventra, Nanotechnology 2013, 24 (41), 415101.

[17] F. Traversi, C. Raillon, S. M. Benameur, K. Liu, S. Khlybov, M. Tosun, D. Krasnozhon, A. Kis, A. Radenovic, Nat. Nanotechnol. 2013, 8 (12), 939 - 945.

[18] M. Aziz, J. Golovchenko, D. Branton, C. McMullan, D. Stein, J. Li, Nature 2001, 412 (6843), 166 - 169.

[19] P. Chen, T. Mitsui, D. B. Farmer, J. Golovchenko, R. G. Gordon, D. Branton, Nano Lett. 2004, 4 (7), 1333 - 1337.

[20] B. M. Venkatesan, B. Dorvel, S. Yemenicioglu, N. Watkins, I. Petrov, R. Bashir, Adv. Mater. 2009, 21 (27), 2771 - 2776.

[21] A. J. Storm, J. H. Chen, X. S. Ling, H. W. Zandbergen, C. Dekker, Nat. Mater. 2003, 2 (8), 537 - 540.

[22] M. Wanunu, T. Dadosh, V. Ray, J. Jin, L. McReynolds, M. Drndi'c, Nat. Nanotechnol. 2010, 5 (11), 807 - 814.

[23] A. T. Kuan, J. A. Golovchenko, Appl. Phys. Lett. 2012, 100 (21), 213104 - 213104-4.

[24] D. J. Niedzwiecki, R. Iyer, P. N. Borer, L. Movileanu, ACS Nano 2013, 7 (4), 3341 - 3350.

[25] Y. Liebes, B. Hadad, N. Ashkenasy, Nanotechnology 2011, 22 (28), 285303.

[26] M.-Y. Wu, D. Krapf, M. Zandbergen, H. Zandbergen, P. E. Batson, Appl. Phys. Lett. 2005, 87, 113106.

[27] M. van den Hout, A. R. Hall, M. Y. Wu, H. W. Zandbergen, C. Dekker, N. H. Dekker, Nanotechnology 2010, 21 (11).

[28] S. Garaj, W. Hubbard, A. Reina, J. Kong, D. Branton, J. Golovchenko, Nature 2010, 467 (7312), 190- 193.

[29] C. A. Merchant, K. Healy, M. Wanunu, V. Ray, N. Peterman, J. Bartel, M. D. Fischbein, K. Venta, Z. T. Luo, A. T. C. Johnson, M. Drndic, Nano Lett. 2010, 10 (8), 2915 - 2921.

[30] G. F. Schneider, S. W. Kowalczyk, V. E. Calado, G. Pandraud, H. W. Zandbergen, L. M. K. Vandersypen, C. Dekker, Nano Lett. 2010, 10 (8), 3163 - 3167.

[31] S. Liu, B. Lu, Q. Zhao, J. Li, T. Gao, Y. B. Chen, Y. F. Zhang, Z. F. Liu, Z. C. Fan, F. H. Yang, L. P. You, D. P. Yu, Adv. Mater. 2013, 25 (33), 4549 - 4554.

[32] R. Wei, T. G. Martin, U. Rant, H. Dietz, Angew. Chem. 2012, 124 (20), 4948 - 4951.

[33] M. Langecker, V. Arnaut, T. G. Martin, J. List, S. Renner, M. Mayer, H. Dietz, F. C. Simmel, Science 2012, 338 (6109), 932 - 936.

[34] N. A. Bell, C. R. Engst, M. Ablay, G. Divitini, C. Ducati, T. Liedl, U. F. Keyser, Nano Lett. 2011, 12 (1), 512 - 517.

[35] J. Shim, J. A. Rivera, R. Bashir, Nanoscale 2013, 5 (22), 10887 - 10893.

[36] J. Larkin, R. Henley, D. C. Bell, T. Cohen-Karni, J. K. Rosenstein, M. Wanunu, ACS Nano 2013, 7 (11), 10121 - 10128.

[37] K. Liu, J. D. Feng, A. Kis, A. Radenovic, ACS Nano 2014, 8 (3), 2504 - 2511.

[38] T. J. Booth, P. Blake, R. R. Nair, D. Jiang, E. W. Hill, U. Bangert, A. Bleloch, M. Gass, K. S. Novoselov, M. I. Katsnelson, A. K. Geim, Nano Lett. 2008, 8 (8), 2442 - 2446.

[39] A. Geim, Nature 2005, 438 (7065), 197 - 200.

[40] A. K. Geim, Science 2009, 324 (5934), 1530 - 1534.

[41] C. Lee, X. Wei, J. W. Kysar, J. Hone, Science 2008, 321 (5887), 385 - 388.

[42] X. Wang, L. Zhi, K. Müllen, Nano Lett. 2008, 8 (1), 323 - 327.

[43] Y. Zhang, Y.-W. Tan, H. L. Stormer, P. Kim, Nature 2005, 438 (7065), 201 - 204.

[44] E. N. Wang, R. Karnik, Nat. Nanotechnol. 2012, 7 (9), 552 - 554.

[45] S. P. Koenig, L. Wang, J. Pellegrino, J. S. Bunch, Nat. Nanotechnol. 2012, 7 (11), 728 - 732.

[46] R. Joshi, P. Carbone, F. Wang, V. Kravets, Y. Su, I. Grigorieva, H. Wu, A. Geim, R. Nair, arXiv preprint arXiv:1401.3134 2014.

[47] K. S. Novoselov, A. K. Geim, S. Morozov, D. Jiang, Y. Zhang, S. Dubonos, I. Grigorieva, A. Firsov, Science 2004, 306 (5696), 666 - 669.

[48] K. Novoselov, D. Jiang, F. Schedin, T. Booth, V. Khotkevich, S. Morozov, A. Geim, Proc. Natl. Acad. Sci. USA 2005, 102 (30), 10451 - 10453.

[49] S. Stankovich, D. A. Dikin, R. D. Piner, K. A. Kohlhaas, A. Kleinhammes, Y. Jia, Y. Wu, S. T. Nguyen, R. S. Ruoff, Carbon 2007, 45 (7), 1558 - 1565.

[50] X. Li, W. Cai, J. An, S. Kim, J. Nah, D. Yang, R. Piner, A. Velamakanni, I. Jung, E. Tutuc, Science 2009, 324 (5932), 1312-1314.

[51] P. Sutter, Nat..Mater 2009, 8 (3), 171 - 172.

[52] S. Garaj, S. Liu, J. A. Golovchenko, D. Branton, Proc. Natl. Acad. Sci. USA 2013, 110 (30), 12192 - 12196.

[53] X. S. Li, Y. W. Zhu, W. W. Cai, M. Borysiak, B. Y. Han, D. Chen, R. D. Piner, L. Colombo, R. S. Ruoff, Nano Lett. 2009, 9 (12), 4359 - 4363.

[54] X. Liang, B. A. Sperling, I. Calizo, G. Cheng, C. A. Hacker, Q. Zhang, Y. Obeng, K. Yan, H. Peng, Q. Li, ACS Nano 2011, 5 (11), 9144- 9153.

[55] W. H. Lin, T. H. Chen, J. K. Chang, J. I. Taur, Y. Y. Lo, W. L. Lee, C. S. Chang, W. B. Su, C. I. Wu, ACS Nano 2014, 8 (2), 1784 - 1791.

[56] B. Alemán, W. Regan, S. Aloni, V. Altoe, N. Alem, C. Girit, B. Geng, L. Maserati, M. Crommie, F. Wang, ACS Nano 2010, 4 (8), 4762 - 4768.

[57] M. J. Kim, M. Wanunu, D. C. Bell, A. Meller, Adv. Mater. 2006, 18 (23), 3149 - 3155.

[58] M. Pimenta, G. Dresselhaus, M. S. Dresselhaus, L. Cancado, A. Jorio, R. Saito, Phys. Chem. Chem. Phys. 2007, 9 (11), 1276 - 1290.

[59] A. C. Ferrari, Solid State Commun. 2007, 143 (1), 47 - 57.

[60] A. Ferrari, J. Meyer, V. Scardaci, C. Casiraghi, M. Lazzeri, F. Mauri, S. Piscanec, D. Jiang, K. Novoselov, S. Roth, Phys. Rev. Lett. 2006, 97 (18), 187401.

[61] K. Yan, H. Peng, Y. Zhou, H. Li, Z. Liu, Nano Lett. 2011, 11 (3), 1106 - 1110.

[62] J.-S. Hwang, Y.-H. Lin, J.-Y. Hwang, R. Chang, S. Chattopadhyay, C.-J. Chen, P. Chen, H.-P. Chiang, T.-R. Tsai, L.-C. Chen, Nanotechnology 2013, 24 (1), 015702.

[63] J. E. Hall, J. Gen. Physiol. 1975, 66 (4), 531 - 532.

[64] S. Wang, Y. Zhang, N. Abidi, L. Cabrales, Langmuir 2009, 25 (18), 11078 - 11081.

[65] B. M. Venkatesan, D. Estrada, S. Banerjee, X. Z. Jin, V. E. Dorgan, M. H. Bae, N. R. Alum, E. Pop, R. Bashir, ACS Nano 2012, 6 (1), 441 - 450.

[66] C. Raillon, P. Granjon, M. Graf, L. J. Steinbock, A. Radenovic, Nanoscale 2012, 4 (16), 4916-4924.

[67] M. Wanunu, J. Sutin, B. McNally, A. Chow, A. Meller, Biophys. J. 2008, 95 (10), 4716 - 4725.

[68] G. F. Schneider, Q. Xu, S. Hage, S. Luik, J. N.H. Spoor, S. Malladi, H. Zandbergen, C. Dekker, Nature Communications, 2013, 4, 2619-2625.

[69] L. S. Connelly, B. Meckes, J. Larkin, A. L. Gillman, M. Wanunu, and R. Lal, Appl. Mater. Interfaces 2014, 6, 5290-5296

**Claims**

1.  A nanopore membrane device comprising:

    (a) a substrate (110) having an upper surface, a lower surface, and an aperture (120), the aperture having one or more walls connecting the upper and lower surfaces and forming a well; and
    (b) a membrane (150) forming a floor of the well, the membrane having a thickness of less than 10 nm and comprising a nanopore (210), wherein the membrane is crack-free such that the background conductance of the membrane is less than 1 nS/$\mu$m$^2$, wherein the membrane device is obtainable by a process comprising the steps of:

    (a) providing the substrate (110);
    (b) depositing a passivating layer (130) on the lower surface of the substrate;
    (c) depositing a sacrificial layer (140) having an upper surface and a lower surface on the passivating layer and across the aperture, thereby forming a floor of the well;
    (d) forming on the upper surface of the sacrificial layer, and optionally also of the passivating layer, the membrane (150), such as a membrane comprising graphene, that extends across the aperture;
    (e) removing the sacrificial layer, leaving the membrane as the floor of the well; and
    (f) creating one or more nanopores (210) in the membrane.

2.  The device of claim 1, wherein the substrate comprises a material selected from the group consisting of silicon nitride, silicon oxide, aluminum oxide, and hafnium oxide.

3.  The device of any one of the preceding claims, wherein the passivating layer comprises material selected from the group consisting of HfO$_2$ and TiO$_2$.

4.  The device of any of the preceding claims, wherein the membrane consists of graphene.

5.  The device of claim 4, wherein the graphene has been chemically stabilized by hydrogenation, hydroxylation, or treatment with a functionalized peptide or surfactant.

6.  The device of claims 4-5, further comprising a plurality of amphiphilic molecules non-covalently bound to a surface of the graphene membrane internal to the well and a surface of each membrane external to the well.

**7.** The device of claims 4-6, further comprising a hydrophilic layer, such as a layer comprising a material selected from the group consisting of $Al_2O_3$, $TiO_2$, $SiO_2$, $HfO_2$, attached to the surface of the graphene membrane external to the well and to the lower surface of the substrate.

**8.** The device of claim 7, further comprising a nanopore in the hydrophilic layer which is aligned with the nanopore in the graphene membrane.

**9.** The device of claims 7-8, further comprising a lipid bilayer non-covalently bound to the hydrophilic layer, the lipid bilayer covering the nanopore.

**10.** The device of claim 9, further comprising a biological nanopore situated in a region of the lipid bilayer covering the nanopore.

**11.** The device of claims 9-10, further comprising a first lipid monolayer non-covalently bound to a surface of the graphene membrane internal to the well and a second lipid monolayer non-covalently bound to a surface of the graphene membrane external to the well, the first and second lipid monolayers covering each nanopore, thereby forming a lipid bilayer spanning each nanopore.

**12.** The device of claims 9-11, further comprising a biological nanopore, such as a biological nanopore comprising alpha-hemolysin, MspA porin, or ClyA porin, disposed in said lipid bilayer.

**13.** An apparatus for sequencing of a polynucleotide, the apparatus comprising:

(a) the device of claims 1-12, wherein the membrane is disposed between a first fluid reservoir and a second fluid reservoir;
(b) an electrode pair comprising a first electrode disposed in the first fluid reservoir and a second electrode disposed in the second fluid reservoir; and
(c) a circuit capable of detecting an electrical signal that correlates with the sequence of the polynucleotide.

**14.** An apparatus for sequencing of a polynucleotide, the apparatus comprising:

(a) the device of claims 4-12, wherein the graphene membrane is disposed between a first fluid reservoir and a second fluid reservoir;
(b) a first electrode pair comprising a first electrode disposed in the first fluid reservoir and a second electrode disposed in the second fluid reservoir, wherein the first electrode pair is capable of applying an electrical field between the fluid reservoirs and across the graphene membrane;
(c) a second electrode pair comprising first and second electrodes in contact with the graphene membrane on opposite sides of the nanopore, wherein the second electrode pair is capable of applying an electrical field laterally through the graphene membrane and across the nanopore of said device; and
(d) a circuit capable of detecting an electrical signal from the second electrode pair that correlates with the sequence of the polynucleotide.

**15.** A method of making a membrane device, the method comprising the steps of:

(a) providing a substrate (110) having an upper surface, a lower surface, and an aperture (120), the aperture having one or more walls connecting the upper and lower surfaces and forming a well;
(b) depositing a passivating layer (130) on the lower surface of the substrate;
(c) depositing a sacrificial layer (140) having an upper surface and a lower surface on the passivating layer and across the aperture, thereby forming a floor of the well;
(d) forming on the upper surface of the sacrificial layer, and optionally also of the passivating layer, a membrane (150), such as a membrane comprising graphene, that extends across the aperture;
(e) removing the sacrificial layer, leaving the membrane as the floor of the well; and
(f) creating one or more nanopores (210) in the membrane.

**16.** A method of determining a sequence of bases of a polynucleotide, the method comprising the steps of:

(a) providing the apparatus of claims 13-14;
(b) adding an aqueous solution comprising the polynucleotide to the first reservoir and adding an aqueous solution to the second reservoir;
(c) applying an electrical field between the first and second electrodes, wherein the electrical field causes one or more charged molecules to transit through the nanopore;
(d) measuring a signal due to the transit of the one or more charged molecules through the nanopore; and
(e) determining the sequence of bases of the polynucleotide from a previously determined correlation between identities of the bases and the signal.

**17.** A method of determining a sequence of bases of a polynucleotide, the method comprising the steps of:

(a) providing the apparatus of claim 14;
(b) adding an aqueous solution comprising the polynucleotide to the first fluid reservoir and adding an aqueous solution to the second fluid reservoir;
(c) applying an electrical field between the first pair of electrodes, wherein the electrical field causes one or more charged molecules to transit through the nanopore;
(d) applying an electrical field between the second pair of electrodes;
(e) measuring a signal between the second pair of electrodes due to the transit of the one or more charged molecules through the nanopore; and
(f) determining the sequence of bases of the polynucleotide from a previously determined correlation between identities of the bases and the signal.

**Patentansprüche**

1. Nanoporen-Membranvorrichtung, umfassend:

   (a) ein Substrat (110), das eine obere Fläche, eine untere Fläche und eine Öffnung (120) aufweist, wobei die Öffnung eine oder mehrere Wände aufweist, die die obere und die untere Fläche verbinden und ein Well bilden; und
   (b) eine Membran (150), die einen Boden des Wells bildet, wobei die Membran eine Dicke von weniger als 10 nm aufweist und eine Nanopore (210) umfasst, wobei die Membran rissfrei ist, sodass die Hintergrundleitfähigkeit der Membran weniger als 1 nS/$\mu$m$^2$ beträgt, wobei die Membranvorrichtung durch einen Prozess erhalten werden kann, der die folgenden Schritte umfasst:

      (a) Bereitstellen des Substrats (110);
      (b) Abscheiden einer Passivierungsschicht (130) auf der unteren Fläche des Substrats;
      (c) Abscheiden einer Opferschicht (140), die eine obere Fläche und eine untere Fläche aufweist, auf der Passivierungsschicht und über der Öffnung, wodurch ein Boden des Wells gebildet wird;
      (d) Bilden der Membran (150) auf der oberen Fläche der Opferschicht und optional auch der Passivierungsschicht , wie eine Membran, die Graphen umfasst, das sich über die Öffnung erstreckt;
      (e) Entfernen der Opferschicht, wobei die Membran als Boden des Wells zurückbleibt; und
      (f) Erzeugen einer oder mehrerer Nanoporen (210) in der Membran.

2. Vorrichtung nach Anspruch 1, wobei das Substrat ein Material umfasst, das ausgewählt ist aus der Gruppe bestehend aus Siliziumnitrid, Siliziumoxid, Aluminiumoxid und Hafniumoxid.

3. Vorrichtung nach einem der vorhergehenden Ansprüche, wobei die Passivierungsschicht ein Material umfasst, das ausgewählt ist aus der Gruppe bestehend aus HfO$_2$ und TiO$_2$.

4. Vorrichtung nach einem der vorhergehenden Ansprüche, wobei die Membran aus Graphen besteht.

5. Vorrichtung nach Anspruch 4, wobei das Graphen durch Hydrierung, Hydroxylierung oder Behandlung mit einem funktionalisierten Peptid oder Tensid chemisch stabilisiert worden ist.

6. Vorrichtung nach Anspruch 4 bis 5, ferner umfassend eine Vielzahl amphiphiler Moleküle, die nicht kovalent an eine Fläche der Graphenmembran im Inneren des Wells und an eine Fläche jeder Membran außerhalb des Wells gebunden sind.

7. Vorrichtung nach Anspruch 4-6, ferner umfassend eine hydrophile Schicht, wie eine Schicht, die ein Material umfasst, das aus der Gruppe ausgewählt ist bestehend aus Al$_2$O$_3$, TiO$_2$, SiO$_2$, HfO$_2$, die an der Fläche der Graphenmembran außerhalb des Wells und an der unteren Fläche des Substrats angebracht ist.

8. Vorrichtung nach Anspruch 7, ferner umfassend eine Nanopore in der hydrophilen Schicht, die auf die Nanopore in der Graphenmembran ausgerichtet ist.

9. Vorrichtung nach Anspruch 7-8, ferner umfassend eine Lipid-Doppelschicht, die nicht kovalent an die hydrophile Schicht gebunden ist, wobei die Lipid-Doppelschicht die Nanopore bedeckt.

10. Vorrichtung nach Anspruch 9, ferner umfassend eine biologische Nanopore, die sich in einem Bereich der Lipid-Doppelschicht befindet, der die Nanopore bedeckt.

11. Vorrichtung nach Anspruch 9-10, ferner umfassend eine erste Lipid-Monoschicht, die nicht kovalent an eine Fläche der Graphenmembran innerhalb des Wells gebunden ist, und eine zweite Lipid-Monoschicht, die nicht kovalent an eine Fläche der Graphenmembran außerhalb des Wells gebunden ist, wobei die erste und die zweite Lipid-Monoschicht jede Nanopore bedecken, wodurch eine Lipid-Doppelschicht gebildet wird, die jede Nanopore überspannt.

12. Vorrichtung nach Anspruch 9-11, ferner umfassend eine biologische Nanopore, wie eine biologische Nanopore, die Alpha-Hämolysin, MspA-Porin oder ClyA-Porin umfasst und in der Lipid-Doppelschicht angeordnet ist.

13. Einrichtung zum Sequenzieren eines Polynukleotids, wobei die Einrichtung folgendes umfasst:

    (a) die Vorrichtung nach Anspruch 1-12, wobei die Membran zwischen einem ersten Fluidreservoir und einem zweiten Fluidreservoir angeordnet ist;

    (b) ein Elektrodenpaar, umfassend eine erste Elektrode, die in dem ersten Fluidreservoir angeordnet ist, und eine zweite Elektrode, die in dem zweiten Fluidreservoir angeordnet ist; und

    (c) eine Schaltung, die in der Lage ist, ein elektrisches Signal zu detektieren, das mit der Sequenz des Polynukleotids korreliert.

14. Einrichtung zum Sequenzieren eines Polynukleotids, wobei die Einrichtung folgendes umfasst:

    (a) die Vorrichtung nach Anspruch 4-12, wobei die Graphenmembran zwischen einem ersten Fluidreservoir und einem zweiten Fluidreservoir angeordnet ist;

    (b) ein erstes Elektrodenpaar, umfassend eine erste Elektrode, die in dem ersten Fluidreservoir angeordnet ist, und eine zweite Elektrode, die in dem zweiten Fluidreservoir angeordnet ist, wobei das erste Elektrodenpaar in der Lage ist, ein elektrisches Feld zwischen den Fluidreservoiren und über die Graphenmembran anzulegen;

    (c) ein zweites Elektrodenpaar, das eine erste und eine zweite Elektrode umfasst, die in Kontakt mit der Graphenmembran auf entgegengesetzten Seiten der Nanopore stehen, wobei das zweite Elektrodenpaar in der Lage ist, ein elektrisches Feld seitlich durch die Graphenmembran und über die Nanopore der Vorrichtung anzulegen; und

    (d) eine Schaltung, die in der Lage ist, ein elektrisches Signal von dem zweiten Elektrodenpaar zu detektieren, das mit der Sequenz des Polynukleotids korreliert.

15. Verfahren zum Herstellen einer Membranvorrichtung, wobei das Verfahren die folgenden Schritte umfasst:

    (a) Bereitstellen eines Substrats (110), das eine obere Fläche, eine untere Fläche und eine Öffnung (120) aufweist, wobei die Öffnung eine oder mehrere Wände aufweist, die die obere und die untere Fläche verbinden und ein Well bilden;

    (b) Abscheiden einer Passivierungsschicht (130) auf der unteren Fläche des Substrats;

    (c) Abscheiden einer Opferschicht (140), die eine obere Fläche und eine untere Fläche aufweist, auf der Passivierungsschicht und über der Öffnung, wodurch ein Boden des Wells gebildet wird;

    (d) Bilden einer Membran (150) auf der oberen Fläche der Opferschicht und optional auch der Passivierungsschicht, wie eine Membran, die Graphen umfasst, das sich über die Öffnung erstreckt;

    (e) Entfernen der Opferschicht, wobei die Membran als Boden des Wells zurückbleibt; und

    (f) Erzeugen einer oder mehrerer Nanoporen (210) in der Membran.

16. Verfahren zum Bestimmen einer Sequenz von Basen eines Polynukleotids, wobei das Verfahren die folgenden Schritte umfasst:

    (a) Bereitstellen der Einrichtung von Anspruch 13-14;

    (b) Zugeben einer wässrigen Lösung, die das Polynukleotid umfasst, zu dem ersten Reservoir und Zugeben einer wässrigen Lösung zu dem zweiten Reservoir;

    (c) Anlegen eines elektrischen Feldes zwischen der ersten und der zweiten Elektrode, wobei das elektrische Feld ein oder mehrere geladene Moleküle veranlasst, durch die Nanopore zu wandern;

    (d) Messen eines Signals, das auf den Durchgang des einen oder der mehreren geladenen Moleküle durch die Nanopore zurückzuführen ist; und

    (e) Bestimmen der Sequenz von Basen des Polynukleotids aus einer zuvor bestimmten Korrelation zwischen den Identitäten der Basen und dem Signal.

17. Verfahren zum Bestimmen einer Sequenz von Basen eines Polynukleotids, wobei das Verfahren die folgenden Schritte umfasst:

    (a) Bereitstellen der Einrichtung von Anspruch 14;

    (b) Zugeben einer wässrigen Lösung, die das Polynukleotid umfasst, zu dem ersten Fluidreservoir und Zugeben einer wässrigen Lösung zu dem zweiten Fluidreservoir;

    (c) Anlegen eines elektrischen Feldes zwischen dem ersten Paar von Elektroden, wobei das elektrische Feld ein oder mehrere geladene Moleküle veranlasst, durch die Nanopore zu wandern;

    (d) Anlegen eines elektrischen Feldes zwischen dem zweiten Paar von Elektroden;

(e) Messen eines Signals zwischen dem zweiten Paar von Elektroden aufgrund des Durchgangs des einen oder der mehreren geladenen Moleküle durch die Nanopore; und

(f) Bestimmen der Sequenz von Basen des Polynukleotids aus einer zuvor bestimmten Korrelation zwischen den Identitäten der Basen und dem Signal.

**Revendications**

1. Dispositif à membrane nanoporeuse comprenant :

(a) un substrat (110) ayant une surface supérieure, une surface inférieure, et une ouverture (120), l'ouverture ayant une ou plusieurs parois reliant les surfaces supérieure et inférieure et formant un puits ; et

(b) une membrane (150) formant un fond du puits, la membrane ayant une épaisseur inférieure à 10 nm et comprenant un nanopore (210), dans lequel la membrane est exempte de fissures de sorte que la conductance de fond de la membrane est inférieure à 1 nS/$\mu$m$^2$, dans lequel le dispositif à membrane peut être obtenu par un procédé comprenant les étapes de :

(a) fourniture du substrat (110) :
(b) dépôt d'une couche de passivation (130) sur la surface inférieure du substrat ;
(c) dépôt d'une couche sacrificielle (140) ayant une surface supérieure et une surface inférieure sur la couche de passivation et à travers l'ouverture, formant ainsi un fond du puits ;
(d) formation sur la surface supérieure de la couche sacrificielle, et éventuellement également de la couche de passivation, de la membrane (150), telle qu'une membrane comprenant du graphène, qui s'étend à travers l'ouverture ;
(e) élimination de la couche sacrificielle, laissant la membrane comme fond du puits ; et
(f) création d'un ou de plusieurs nanopores (210) dans la membrane.

2. Dispositif selon la revendication 1, dans lequel le substrat comprend un matériau choisi dans le groupe constitué de nitrure de silicium, d'oxyde de silicium, d'oxyde d'aluminium et d'oxyde d'hafnium.

3. Dispositif selon l'une quelconque des revendications précédentes, dans lequel la couche de passivation comprend un matériau choisi dans le groupe constitué de HfO$_2$ et TiO$_2$.

4. Dispositif selon l'une quelconque des revendications précédentes, dans lequel la membrane est constituée de graphène.

5. Dispositif selon la revendication 4, dans lequel le graphène a été chimiquement stabilisé par hydrogénation, hydroxylation ou traitement par un peptide ou un tensioactif fonctionnalisé.

6. Dispositif selon les revendications 4 et 5, comprenant en outre une pluralité de molécules amphiphiles liées de manière non covalente à une surface de la membrane de graphène à l'intérieur du puits et à une surface de chaque membrane à l'extérieur du puits.

7. Dispositif selon les revendications 4 à 6, comprenant en outre une couche hydrophile, telle qu'une couche comprenant un matériau choisi dans le groupe constitué de Al$_2$O$_3$, TiO$_2$, SiO$_2$, HfO$_2$, fixée à la surface de la membrane de graphène à l'extérieur du puits et à la surface inférieure du substrat.

8. Dispositif selon la revendication 7, comprenant en outre un nanopore dans la couche hydrophile qui est aligné avec le nanopore dans la membrane de graphène.

9. Dispositif selon les revendications 7 et 8, comprenant en outre une bicouche lipidique liée de manière non covalente à la couche hydrophile, la bicouche lipidique recouvrant le nanopore.

10. Dispositif selon la revendication 9, comprenant en outre un nanopore biologique situé dans une région de la bicouche lipidique recouvrant le nanopore.

11. Dispositif selon les revendications 9 et 10, comprenant en outre une première monocouche lipidique liée de manière non covalente à une surface de la membrane de graphène à l'intérieur du puits et une seconde monocouche lipidique liée de manière non covalente à une surface de la membrane de graphène à l'extérieur du puits, les première et seconde monocouches lipidiques recouvrant chaque nanopore, formant ainsi une bicouche lipidique couvrant chaque nanopore.

12. Dispositif selon les revendications 9 à 11, comprenant en outre un nanopore biologique, tel qu'un nanopore biologique comprenant de l'alpha-hémolysine, de la porine MspA ou de la porine ClyA, disposé dans ladite bicouche lipidique.

13. Appareil pour le séquençage d'un polynucléotide, l'appareil comprenant :

(a) le dispositif selon les revendications 1 à 12, dans lequel la membrane est disposée entre un premier réservoir de fluide et un second réservoir de fluide ;

(b) une paire d'électrodes comprenant une première électrode disposée dans le premier réservoir de fluide et une seconde électrode disposée dans le second réservoir de fluide ; et

(c) un circuit capable de détecter un signal électrique qui est en corrélation avec la séquence du polynucléotide.

14. Appareil pour le séquençage d'un polynucléotide, l'appareil comprenant :

(a) le dispositif selon les revendications 4 à 12, dans lequel la membrane de graphène est disposée entre un premier réservoir de fluide et un second réservoir de fluide ;

(b) une première paire d'électrodes comprenant une première électrode disposée dans le premier réservoir de fluide et une seconde électrode disposée dans le second réservoir de fluide, dans lequel la première paire d'électrodes est capable d'appliquer un champ électrique entre les réservoirs de fluide et à travers la membrane de graphène ;

(c) une seconde paire d'électrodes comprenant des première et seconde électrodes en contact avec la membrane de graphène sur des côtés opposés du nanopore, dans lequel la seconde paire d'électrodes est capable d'appliquer un champ électrique latéralement à travers la membrane de graphène et à travers le nanopore dudit dispositif ; et

(d) un circuit capable de détecter un signal électrique provenant de la seconde paire d'électrodes qui est en corrélation avec la séquence du polynucléotide.

15. Procédé de fabrication d'un dispositif à membrane, le procédé comprenant les étapes de :

(a) fourniture d'un substrat (110) ayant une surface supérieure, une surface inférieure et une ouverture (120), l'ouverture ayant une ou plusieurs parois reliant les surfaces supérieure et inférieure et formant un puits ;

(b) dépôt d'une couche de passivation (130) sur la surface inférieure du substrat ;

(c) dépôt d'une couche sacrificielle (140) ayant une surface supérieure et une surface inférieure sur la couche de passivation et à travers l'ouverture, formant ainsi un fond du puits ;

(d) formation sur la surface supérieure de la couche sacrificielle, et éventuellement également de la couche de passivation, d'une membrane (150), telle qu'une membrane comprenant du graphène, qui s'étend à travers l'ouverture ;

(e) élimination de la couche sacrificielle, laissant la membrane comme fond du puits ; et

(f) création d'un ou de plusieurs nanopores (210) dans la membrane.

16. Procédé de détermination d'une séquence de bases d'un polynucléotide, le procédé comprenant les étapes de :

(a) fourniture de l'appareil selon les revendications 13 et 14 ;

(b) ajout d'une solution aqueuse comprenant le polynucléotide au premier réservoir et ajout d'une solution aqueuse au second réservoir ;

(c) application d'un champ électrique entre les première et seconde électrodes, dans lequel le champ électrique fait transiter une ou plusieurs molécules chargées à travers le nanopore ;

(d) mesure d'un signal dû au transit de la ou des molécules chargées à travers le nanopore ; et

(e) détermination de la séquence de bases du polynucléotide à partir d'une corrélation préalablement déterminée entre les identités des bases et le signal.

17. Procédé de détermination d'une séquence de bases d'un polynucléotide, le procédé comprenant les étapes de :

(a) fourniture de l'appareil selon la revendication 14 ;

(b) ajout d'une solution aqueuse comprenant le polynucléotide au premier réservoir de fluide et ajout d'une solution aqueuse au second réservoir de fluide ;

(c) application d'un champ électrique entre la première paire d'électrodes, dans lequel le champ électrique fait transiter une ou plusieurs molécules chargées à travers le nanopore ;

(d) application d'un champ électrique entre la seconde paire d'électrodes ;

(e) mesure d'un signal entre la seconde paire d'électrodes dû au transit de la ou des molécules chargées à travers le nanopore ; et

(f) détermination de la séquence de bases du polynucléotide à partir d'une corrélation préalablement déterminée entre les identités des bases et le signal.

**FIG. 1**

EP 3 074 551 B1

FIG. 2

FIG. 3

**FIG. 4**

A

B

C

D

FIG. 5

**FIG. 6**

**FIG. 7**

FIG. 8

**FIG. 9**

EP 3 074 551 B1

## REFERENCES CITED IN THE DESCRIPTION

### Patent documents cited in the description

- US 61908695
- US 2013309776 A
- WO 2013185137 A
- US 8652779 B
- US 20130264207 A
- WO 2012088339 A
- US 20070190542 A
- WO 2013119784 A
- US 20130256118 A

### Non-patent literature cited in the description

- **V. BERRY.** *Carbon,* 2013, vol. 62, 1-10
- **H. W. KIM ; H. W. YOON ; S.-M. YOON ; B. M. YOO ; B. K. AHN ; Y. H. CHO ; H. J. SHIN ; H. YANG ; U. PAIK ; S. KWON.** *Science,* 2013, vol. 342 (6154), 91-95
- **R. NAIR ; H. WU ; P. JAYARAM ; I. GRIGORIEVA ; A. GEIM.** *Science,* 2012, vol. 335 (6067), 442-444
- **A. W. HAUSER ; P. SCHWERDTFEGER.** *J. Phys. Chem. Lett.,* 2012, vol. 3 (2), 209-213
- **L. W. DRAHUSHUK ; M. S. STRANO.** *Langmuir,* 2012, vol. 28 (48), 16671-16678
- **D.-E. JIANG ; V. R. COOPER ; S. DAI.** *Nano Lett.,* 2009, vol. 9 (12), 4019-4024
- **S. C. O'HERN ; C. A. STEWART ; M. S. BOUTILIER ; J.-C. IDROBO ; S. BHAVIRIPUDI ; S. K. DAS ; J. KONG ; T. LAOUI ; M. ATIEH ; R. KARNIK.** *ACS Nano,* 2012, vol. 6 (11), 10130-10138
- **H. DU ; J. LI ; J. ZHANG ; G. SU ; X. LI ; Y. ZHAO ; J. PHYS.** *Chem. C,* 2011, vol. 115 (47), 23261-23266
- **L. S. CONNELLY ; B. MECKES ; J. LARKIN ; A. L. GILLMAN ; M. WANUNU ; R. LAL.** *ACS App. Mat. Int.,* 2014, vol. 6 (7), 5290-5296
- **A. D. SMITH ; F. NIKLAUS ; A. PAUSSA ; S. VAZIRI ; A. C. FISCHER ; M. STERNER ; F. FORSBERG ; A. DELIN ; D. ESSENI ; P. PALESTRI.** *Nano Lett.,* 2013, vol. 13 (7), 3237-3242
- **M. LIAO ; Y. KOIDE.** *Critical Rev. Solid State Mater. Sci.,* 2011, vol. 36 (2), 66-101
- **S. BASU ; P. BHATTACHARYYA.** *Sens. Actuat. B: Chem.,* 2012, vol. 173, 1-21
- **T. MIAO ; S. YEOM ; P. WANG ; B. STANDLEY ; M. BOCKRATH.** *Nano Lett.,* 2014
- **K. K. SAHA ; M. DRNDIC ; B. K. NIKOLIC.** *Nano Lett.,* 2012, vol. 12 (1), 50-55
- **M. PUSTER ; J. A. RODRIGUEZ-MANZO ; A. BALAN ; M. DRNDIC.** *ACS Nano,* 2013, vol. 7 (12), 11283-11289
- **J. WILSON ; M. DI VENTRA.** *Nanotechnology,* 2013, vol. 24 (41), 415101
- **F. TRAVERSI ; C. RAILLON ; S. M. BENAMEUR ; K. LIU ; S. KHLYBOV ; M. TOSUN ; D. KRASNOZHON ; A. KIS ; A. RADENOVIC.** *Nat. Nanotechnol.,* 2013, vol. 8 (12), 939-945
- **M. AZIZ ; J. GOLOVCHENKO ; D. BRANTON ; C. MCMULLAN ; D. STEIN ; J. LI.** *Nature,* 2001, vol. 412 (6843), 166-169
- **P. CHEN ; T. MITSUI ; D. B. FARMER ; J. GOLOVCHENKO ; R. G. GORDON ; D. BRANTON.** *Nano Lett.,* 2004, vol. 4 (7), 1333-1337
- **B. M. VENKATESAN ; B. DORVEL ; S. YEMENICIOGLU ; N. WATKINS ; I. PETROV ; R. BASHIR.** *Adv. Mater.,* 2009, vol. 21 (27), 2771-2776
- **A. J. STORM ; J. H. CHEN ; X. S. LING ; H. W. ZANDBERGEN ; C. DEKKER.** *Nat. Mater.,* 2003, vol. 2 (8), 537-540
- **M. WANUNU ; T. DADOSH ; V. RAY ; J. JIN ; L. MCREYNOLDS ; M. DRNDI'C.** *Nat. Nanotechnol.,* 2010, vol. 5 (11), 807-814
- **A. T. KUAN ; J. A. GOLOVCHENKO.** *Appl. Phys. Lett.,* 2012, vol. 100 (21), 213104-213104
- **D. J. NIEDZWIECKI ; R. IYER ; P. N. BORER ; L. MOVILEANU.** *ACS Nano,* 2013, vol. 7 (4), 3341-3350
- **Y. LIEBES ; B. HADAD ; N. ASHKENASY.** *Nanotechnology,* 2011, vol. 22 (28), 285303
- **M.-Y. WU ; D. KRAPF ; M. ZANDBERGEN ; H. ZANDBERGEN ; P. E. BATSON.** *Appl. Phys. Lett.,* 2005, vol. 87, 113106
- **M. VAN DEN HOUT ; A. R. HALL ; M. Y. WU ; H. W. ZANDBERGEN ; C. DEKKER ; N. H. DEKKER.** *Nanotechnology,* 2010, vol. 21 (11

- **S. GARAJ ; W. HUBBARD ; A. REINA ; J. KONG ; D. BRANTON ; J. GOLOVCHENKO.** *Nature,* 2010, vol. 467 (7312), 190-193
- **C. A. MERCHANT ; K. HEALY ; M. WANUNU ; V. RAY ; N. PETERMAN ; J. BARTEL ; M. D. FISCHBEIN ; K. VENTA ; Z. T. LUO ; A. T. C. JOHNSON.** *Nano Lett.,* 2010, vol. 10 (8), 2915-2921
- **G. F. SCHNEIDER ; S. W. KOWALCZYK ; V. E. CALADO ; G. PANDRAUD ; H. W. ZANDBERGEN ; L. M. K. VANDERSYPEN ; C. DEKKER.** *Nano Lett.,* 2010, vol. 10 (8), 3163-3167
- **S. LIU ; B. LU ; Q. ZHAO ; J. LI ; T. GAO ; Y. B. CHEN ; Y. F. ZHANG ; Z. F. LIU ; Z. C. FAN ; F. H. YANG.** *Adv. Mater.,* 2013, vol. 25 (33), 4549-4554
- **R. WEI ; T. G. MARTIN ; U. RANT ; H. DIETZ.** *Angew. Chem.,* 2012, vol. 124 (20), 4948-4951
- **M. LANGECKER ; V. ARNAUT ; T. G. MARTIN ; J. LIST ; S. RENNER ; M. MAYER ; H. DIETZ ; F. C. SIMMEL.** *Science,* 2012, vol. 338 (6109), 932-936
- **N. A. BELL ; C. R. ENGST ; M. ABLAY ; G. DIVITINI ; C. DUCATI ; T. LIEDL ; U. F. KEYSER.** *Nano Lett.,* 2011, vol. 12 (1), 512-517
- **J. SHIM ; J. A. RIVERA ; R. BASHIR.** *Nanoscale,* 2013, vol. 5 (22), 10887-10893
- **J. LARKIN ; R. HENLEY ; D. C. BELL ; T. COHEN-KARNI ; J. K. ROSENSTEIN ; M. WANUNU.** *ACS Nano,* 2013, vol. 7 (11), 10121-10128
- **K. LIU ; J. D. FENG ; A. KIS ; A. RADENOVIC.** *ACS Nano,* 2014, vol. 8 (3), 2504-2511
- **T. J. BOOTH ; P. BLAKE ; R. R. NAIR ; D. JIANG ; E. W. HILL ; U. BANGERT ; A. BLELOCH ; M. GASS ; K. S. NOVOSELOV ; M. I. KATSNELSON.** *Nano Lett.,* 2008, vol. 8 (8), 2442-2446
- **A. GEIM.** *Nature,* 2005, vol. 438 (7065), 197-200
- **A. K. GEIM.** *Science,* 2009, vol. 324 (5934), 1530-1534
- **C. LEE ; X. WEI ; J. W. KYSAR ; J. HONE.** *Science,* 2008, vol. 321 (5887), 385-388
- **X. WANG ; L. ZHI ; K. MÜLLEN.** *Nano Lett.,* 2008, vol. 8 (1), 323-327
- **Y. ZHANG ; Y.-W. TAN ; H. L. STORMER ; P. KIM.** *Nature,* 2005, vol. 438 (7065), 201-204
- **E. N. WANG ; R. KARNIK.** *Nat. Nanotechnol.,* 2012, vol. 7 (9), 552-554
- **S. P. KOENIG ; L. WANG ; J. PELLEGRINO ; J. S. BUNCH.** *Nat. Nanotechnol.,* 2012, vol. 7 (11), 728-732
- **R. JOSHI ; P. CARBONE ; F. WANG ; V. KRAVETS ; Y. SU ; I. GRIGORIEVA ; H. WU ; A. GEIM ; R. NAIR.** *arXiv:1401.3134,* 2014
- **K. S. NOVOSELOV ; A. K. GEIM ; S. MOROZOV ; D. JIANG ; Y. ZHANG ; S. DUBONOS ; I. GRIGORIEVA ; A. FIRSOV.** *Science,* 2004, vol. 306 (5696), 666-669
- **K. NOVOSELOV ; D. JIANG ; F. SCHEDIN ; T. BOOTH ; V. KHOTKEVICH ; S. MOROZOV ; A. GEIM.** *Proc. Natl. Acad. Sci. USA,* 2005, vol. 102 (30), 10451-10453
- **S. STANKOVICH ; D. A. DIKIN ; R. D. PINER ; K. A. KOHLHAAS ; A. KLEINHAMMES ; Y. JIA ; Y. WU ; S. T. NGUYEN ; R. S. RUOFF.** *Carbon,* 2007, vol. 45 (7), 1558-1565
- **X. LI ; W. CAI ; J. AN ; S. KIM ; J. NAH ; D. YANG ; R. PINER ; A. VELAMAKANNI ; I. JUNG ; E. TUTUC.** *Science,* 2009, vol. 324 (5932), 1312-1314
- **P. SUTTER.** *Nat..Mater,* 2009, vol. 8 (3), 171-172
- **S. GARAJ ; S. LIU ; J. A. GOLOVCHENKO ; D. BRANTON.** *Proc. Natl. Acad. Sci. USA,* 2013, vol. 110 (30), 12192-12196
- **X. S. LI ; Y. W. ZHU ; W. W. CAI ; M. BORYSIAK ; B. Y. HAN ; D. CHEN ; R. D. PINER ; L. COLOMBO ; R. S. RUOFF.** *Nano Lett.,* 2009, vol. 9 (12), 4359-4363
- **X. LIANG ; B. A. SPERLING ; I. CALIZO ; G. CHENG ; C. A. HACKER ; Q. ZHANG ; Y. OBENG ; K. YAN ; H. PENG ; Q. LI.** *ACS Nano,* 2011, vol. 5 (11), 9144-9153
- **W. H. LIN ; T. H. CHEN ; J. K. CHANG ; J. I. TAUR ; Y. Y. LO ; W. L. LEE ; C. S. CHANG ; W. B. SU ; C. I. WU.** *ACS Nano,* 2014, vol. 8 (2), 1784-1791
- **B. ALEMÁN ; W. REGAN ; S. ALONI ; V. ALTOE ; N. ALEM ; C. GIRIT ; B. GENG ; L. MASERATI ; M. CROMMIE ; F. WANG.** *ACS Nano,* 2010, vol. 4 (8), 4762-4768
- **M. J. KIM ; M. WANUNU ; D. C. BELL ; A. MELLER.** *Adv. Mater.,* 2006, vol. 18 (23), 3149-3155
- **M. PIMENTA ; G. DRESSELHAUS ; M. S. DRESSELHAUS ; L. CANCADO ; A. JORIO ; R. SAITO.** *Phys. Chem. Chem. Phys.,* 2007, vol. 9 (11), 1276-1290
- **A. C. FERRARI.** *Solid State Commun.,* 2007, vol. 143 (1), 47-57
- **A. FERRARI ; J. MEYER ; V. SCARDACI ; C. CASIRAGHI ; M. LAZZERI ; F. MAURI ; S. PISCANEC ; D. JIANG ; K. NOVOSELOV ; S. ROTH.** *Phys. Rev. Lett.,* 2006, vol. 97 (18), 187401
- **K. YAN ; H. PENG ; Y. ZHOU ; H. LI ; Z. LIU.** *Nano Lett.,* 2011, vol. 11 (3), 1106-1110
- **J.-S. HWANG ; Y.-H. LIN ; J.-Y. HWANG ; R. CHANG ; S. CHATTOPADHYAY ; C.-J. CHEN ; P. CHEN ; H.-P. CHIANG ; T.-R. TSAI ; L.-C. CHEN.** *Nanotechnology,* 2013, vol. 24 (1), 015702
- **J. E. HALL.** *J. Gen. Physiol.,* 1975, vol. 66 (4), 531-532
- **S. WANG ; Y. ZHANG ; N. ABIDI ; L. CABRALES.** *Langmuir,* 2009, vol. 25 (18), 11078-11081

- **B. M. VENKATESAN ; D. ESTRADA ; S. BANERJEE ; X. Z. JIN ; V. E. DORGAN ; M. H. BAE ; N. R. ALUM ; E. POP ; R. BASHIR.** *ACS Nano,* 2012, vol. 6 (1), 441-450
- **C. RAILLON ; P. GRANJON ; M. GRAF ; L. J. STEINBOCK ; A. RADENOVIC.** *Nanoscale,* 2012, vol. 4 (16), 4916-4924
- **M. WANUNU ; J. SUTIN ; B. MCNALLY ; A. CHOW ; A. MELLER.** *Biophys. J.,* 2008, vol. 95 (10), 4716-4725
- **G. F. SCHNEIDER ; Q. XU ; S. HAGE ; S. LUIK ; J. N.H. SPOOR ; S. MALLADI ; H. ZANDBERGEN ; C. DEKKER.** *Nature Communications,* 2013, vol. 4, 2619-2625
- **L. S. CONNELLY ; B. MECKES ; J. LARKIN ; A. L. GILLMAN ; M. WANUNU ; R. LAL.** *Appl. Mater. Interfaces,* 2014, vol. 6, 5290-5296